(19) **Europäisches Patentamt / European Patent Office / Office européen des brevets**

(11) **EP 1 784 423 B1**

(12) **FASCICULE DE BREVET EUROPEEN**

(45) Date de publication et mention
de la délivrance du brevet:
**08.10.2008 Bulletin 2008/41**

(21) Numéro de dépôt: **05793330.1**

(22) Date de dépôt: **29.07.2005**

(51) Int Cl.:
*C07K 14/78* ^(2006.01)    *A61K 38/39* ^(2006.01)

(86) Numéro de dépôt international:
**PCT/FR2005/001999**

(87) Numéro de publication internationale:
**WO 2006/018551 (23.02.2006 Gazette 2006/08)**

(54) **NOUVEAU PEPTIDE ET COMPOSITION PHARMACEUTIQUE LE CONTENANT**

NEUES PEPTID UND PHARMAZEUTISCHE ZUSAMMENSETZUNG, DIE DIESES PEPTID ENTHÄLT

NOVEL PEPTIDE AND PHARMACEUTICAL COMPOSITION CONTAINING SAID PEPTIDE

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **29.07.2004 FR 0408383**

(43) Date de publication de la demande:
**16.05.2007 Bulletin 2007/20**

(73) Titulaires:
• **CENTRE NATIONAL DE
LA RECHERCHE SCIENTIFIQUE (CNRS)
75794 Paris Cedex 16 (FR)**
• **Laboratoires d'Anjou
75007 Paris (FR)**

(72) Inventeur: **ROUSSELLE, Patricia
F-69230 Saint Genis Laval (FR)**

(74) Mandataire: **Wagret, Frédéric
Cabinet Wagret
19, rue de Milan
75009 Paris (FR)**

(56) Documents cités:
**WO-A-00/66731        US-B1- 6 294 356**

**EP 1 784 423 B1**

LISTE DE SEQUENCES

<110> Laboratoires d'Anjou
Centre National de la Recherche Scientifique - CNRS

<120> Nouveau peptide et composition pharmaceutique le contenant.

<130> 348154/D22277

<150> FR 04/08383
<151> 2004-07-29

<160> 3

<170> Patentln Ver. 2.1

<210> 1
<211> 12
<212> PRT
<213> Laminine 5 humaine.

<400> 1

```
Thr Ala Leu Arg Ile Arg Ala Thr Tyr Gly Glu Tyr
1               5                   10
```

<210> 2
<211> 12
<212> PRT
<213> Laminine 5 humaine.

<400> 2

```
        Gly Leu Thr Lys Thr Tyr Thr Phe Arg Leu Asn Glu
         1               5                   10
```

<210> 3
<211> 12
<212> PRT
<213> Laminine 5 humaine.

<400> 3

```
        Asp Val Lys Asn Leu Glu Asn 1le Arg Asp Asn Leu
         1               5                   10
```

**Description**

La présente invention se rapporte à un nouveau peptide, à une composition pharmaceutique comprenant ce peptide et à l'utilisation d'une telle composition pour le traitement des altérations de la peau d'origines diverses.

Plus particulièrement, le traitement desdites altérations consiste notamment à renforcer la jonction dermo-épidermique, l'adhérence cellule-cellule et/ou l'adhérence cellule-matrice au niveau de l'épiderme et à favoriser la réparation de la surface cutanée.

L'invention se rapporte encore à un procédé de traitement cosmétique comprenant l'application sur la peau de cette composition pharmaceutique.

L'altération de la peau la plus commune et la plus naturelle est tout simplement celle due au vieillissement cutané.

Le vieillissement cutané est un phénomène complexe qui est dû à de nombreux facteurs intrinsèques (facteurs génétiques) et extrinsèques (tels que le soleil, l'alimentation, l'exposition à la fumée de cigarette...). Cliniquement, on observe l'apparition de rides et de ridules, une perte de l'élasticité cutanée, un relâchement des tissus cutanés et sous-cutanés ainsi qu'une moins bonne cicatrisation.

De nombreuses voies de recherches sont proposées pour lutter contre le vieillissement cutané, parmi lesquelles la protection contre l'environnement (soleil, pollutions...), l'activation de la régénération cellulaire, le renforcement de la matrice extracellulaire (collagène et élastine). Récemment, des études ont montré l'importance de l'adhérence des cellules entre elles d'une part, et de l'adhérence entre les cellules et la matrice extracellulaire d'autre part, dans le processus du vieillissement cutané et donc de la nécessité à les renforcer pour prévenir voire traiter le relâchement de la peau.

Les études dermatologiques récemment tournées vers l'utilisation des peptides en biologie cutanée (séquences dérivées de l'alpha-MSH, certains neuropeptides, peptide RGD...), s'orientent vers la recherche de peptides ayant une activité forte au niveau cutané.

L'industrie cosmétique est également dans l'attente permanente d'un nouveau peptide capable d'augmenter l'adhérence des cellules à la matrice extracellulaire et/ou l'adhérence des cellules entre elles.

Les interactions entre les cellules et la matrice extracellulaire (MEC) jouent en effet un rôle important dans le contrôle du comportement cellulaire. Ce contrôle s'effectue par des interactions spécifiques entre les molécules matricielles et les cellules au niveau de récepteurs transmembranaires, principalement de la famille des intégrines, dont le domaine intracellulaire est relié aux constituants du cytosquelette. Ceci permet à la matrice d'assurer la transmission de signaux intracellulaires modulant selon les cas l'adhérence, la migration, la prolifération, la différentiation ou l'apoptose des cellules de l'épiderme. Ce contrôle du comportement cellulaire est crucial au cours du développement mais également lors des remaniements tissulaires.

Selon les molécules constitutives et l'organisation qui en découle, on distingue plusieurs types de MEC, les lames basales étant sans doute les plus spécialisées. Ces dernières sont de fins treillis protéiques continus sur lesquels reposent les différents feuillets cellulaires de l'organisme. Elles ont longtemps été définies comme des structures morphologiques discrètes dont la fonction était limitée au cloisonnement des compartiments tissulaires. C'est seulement au cours des 15 dernières années que, malgré leur faible représentativité et leur grande insolubilité, des avancées significatives ont été réalisées dans l'investigation de leur composition moléculaire et de leurs fonctions. Il apparaît que ces structures jouent un rôle fondamental dans le contrôle du comportement cellulaire, tant au cours du développement embryonnaire que dans le maintien de l'intégrité des phénotypes cellulaires différenciés.

La structure de la peau est composée :

-   d'un épithélium de revêtement : l'épiderme,
-   d'un tissu conjonctif : le derme (couche épaisse déterminant la morphologie de la peau et contenant les vaisseaux sanguins et lymphatiques, les nerfs), et
-   d'un tissu adipeux : l'hypoderme.

Derme et épiderme sont reliés par une structure unique et complexe, la jonction dermo-épidermique (JDE) ou lame basale épidermique. Anatomiquement, elle correspond à la zone comprise entre les cellules basales de l'épiderme et les couches les plus superficielles du derme. C'est une zone d'adhérence qui détermine un cloisonnement entre l'épithélium polarisé et le stroma sous-jacent, assurant le maintien et la cohésion des cellules adjacentes. La JDE, composée exclusivement de matrice extracellulaire, assure deux rôles :

   1°) rôle mécanique,
   son architecture moléculaire unique lui confère une grande stabilité mécanique lui permettant d'assurer l'ancrage solide de l'épiderme ;
   2°) rôle biologique,
   les protéines de la JDE entretiennent avec les cellules basales de l'épiderme des relations étroites et leur transmettent des informations morphogénétiques importantes par l'intermédiaire des récepteurs de la famille des intégrines.

La JDE est aussi un filtre de diffusion vis-à-vis des éléments nutritifs et métaboliques. Elle permet donc la transmission des informations biologiques.

Au cours du vieillissement cutané, la JDE s'aplanit et perd progressivement ses ondulations caractéristiques, ce qui réduit considérablement l'interface épiderme-derme. De plus, les réseaux moléculaires se désorganisent, la charpente protéique se fragilise et l'adhérence des kératinocytes basaux est amoindrie.

En conséquence, la fonction mécanique (soutien) et la fonction biologique (échange d'informations et de molécules) de la JDE sont altérées.

Lorsqu'il y a blessure cutanée, la JDE est endommagée et ses molécules constitutives sont dégradées par des enzymes spécifiques. Dans des conditions favorables, la réépithélisation épidermique débute quelques heures après le traumatisme. Dès que l'épithélium a recouvert le lit de la plaie, les protéines de la JDE réapparaissent de façon séquentielle et ordonnée.

Avec le vieillissement cutané, chacune de ces étapes se déroule plus lentement. En particulier, il a été observé une diminution de l'expression des protéines d'adhérence matricielle ainsi que de celle des récepteurs membranaires, qui pourrait constituer la cause majeure du retard observé dans le processus de reconstitution de la JDE et de l'extrême fragilité des tissus cicatrisés chez les sujets âgés.

La laminine 5 (LN-5) est une protéine spécifiquement exprimée dans les lames basales des épithélia squameux et transitionnels spécialisés comme la jonction dermo-épidermique de la peau. La LN-5 résulte de l'assemblage hétérotrimérique des sous-unités alpha 3, bêta 3 et gamma 2 et est synthétisée exclusivement par les cellules épithéliales sous la forme d'un précurseur de 460 kDa. Dans les conditions physiologiques, chacune des sous-unités a3 et gamma 2 subit un clivage post-traductionnel extracellulaire des extrémités carboxy-et amino-terminales respectivement, aboutissant à la forme tissulaire mature. Des études récentes indiquent que la totalité de la chaîne gamma 2 précurseur est nécessaire à la sécrétion à l'intégration de la LN-5 dans la MEC.

Le rôle majeur de la LN-5 est souligné par l'existence de pathologies héréditaires ou acquises, résultant d'une anomalie de synthèse et/ou d'expression de l'une de ses sous-unités constitutives. Ces maladies, appelées épidermolyses bulleuses jonctionnelles, conduisent notamment à une fragilité de la jonction dermo-épidermique de la peau caractérisée par la formation spontanée de bulles épidermiques. La LN-5 joue ainsi un rôle crucial et irremplaçable dans la cohésion épithélio-mésenchymateuse. La LN-5 est porteuse de signaux biologiques déterminants puisqu'elle permet l'adhérence des cellules épithéliales adjacentes par l'intermédiaire des intégrines et induit l'assemblage des structures d'adhérence stable que sont les hémidesmosomes.

La demande de brevet internationale WO 00/66731 au nom de Biostatum décrit la production de LN-5 entière sous forme recombinante dans des cellules eucaryotes (L5r) et documente son activité pour améliorer la cicatrisation, la prolifération et l'adhérence cellulaire. D'autres peptides derivés de la Laminine-5 sont décrits en US-B1-6294356 (Jones).

Or, la demanderesse a trouvé, de façon surprenante et inattendue, qu'une quantité efficace d'un peptide de séquence TALRIRATYGEY, séquence présente sur la chaîne gamma 2 de la LN-5, induit de façon spécifique l'adhérence des kératinocytes de l'épiderme et d'autres cellules épithéliales. Ce peptide permet non seulement d'augmenter l'adhérence des cellules à la MEC mais également d'augmenter l'adhérence des cellules entre elles. De par sa petite taille et sa grande stabilité, le peptide présente toutes les caractéristiques requises pour traverser convenablement l'épiderme et atteindre sa cible, la JDE, et interagir avec les kératinocytes basaux et transmettre des signaux d'induction d'adhérence. Fragment de la LN-5, il permettra de restaurer son homologue natif déficient ou manquant et aura une activité biologique immédiate d'induction de l'adhérence et de restauration des propriétés mécaniques de la JDE. Son procédé de fabrication par synthèse chimique est simple et applicable à l'échelle industrielle. Il ne fait pas appel à l'utilisation de cultures de cellules d'origine animale, ni à des facteurs de croissance et/ou de sérums ou dérivés de sérums. De petite taille, il sera peu ou pas la cible de dégradations protéolytiques spécifiques et/ou non spécifiques.

Ainsi, l'invention a pour premier objet un peptide présentant la séquence suivante : TALRIRATYGEY (SEQ ID N°1).

La présente invention a également pour objet d'autres peptides résultant d'une ou plusieurs modification(s) de SEQ ID N° 1 telles que l'ajout, la suppression ou la substitution d'un ou plusieurs acides aminés, de préférence comme indiqué dans le tableau 1, et/ou d'une oligomérisation, d'une cyclisation ou du repliement de SEQ ID N° 1, étant entendu que ces modifications ne diminuent en rien l'activité adhésive du peptide de référence, voire l'améliorent.

Tableau 1

| Position des acides aminés | SEQ ID N° 1 | Autres peptides selon l'invention résultant d'une ou plusieurs des substances suivantes |
|---|---|---|

(suite)

| 1 | T | X* |
|---|---|---|
| 2 | A | X* |
| 3 | L | L,M,I,V,F,EouR |
| 4 | R | R, N, E, Q, H, K ou Y |
| 5 | I | I,M,L,V,F,T ou K |
| 6 | R | R, N, E, Q, H, K ou T |
| 7 | A | A, C, S, G, V ou D |
| 8 | T | T,S,P,G,N,D,E,Q,HouK |
| 9 | Y | Y, H, F, W, D, I ou Q |
| 10 | G | G, S, T, A ou N |
| 11 | E | X* |
| 12 | Y | X* |

*X pouvant être n'importe quel acide aminé.

Plus particulièrement, l'ajout ou le retrait d'un ou plusieurs acides aminés peut être réalisé du côté carboxy et /ou du coté amino-terminal dudit peptide. L'invention a également pour objet tout analogue ou dérivé qui résulterait de la greffe d'un motif d'intérêt (molécules naturelles ou synthèse, protéines et/ou sucres) sur ledit peptide. Elle a également pour objet tout fragment dermatologiquement actif du peptide de l'invention, modifié ou non.

Le peptide selon la présente invention est caractérisé en ce qu'il est obtenu par la synthèse chimique.

L'invention a également pour objet une composition pharmaceutique caractérisée en ce qu'elle comprend au moins un peptide selon la présente invention.

Par « composition pharmaceutique », on entend toute composition dermatologique apte à être utilisée à des fins cosmétiques et/ou à des fins thérapeutiques.

Selon un mode de réalisation avantageux de l'invention, le susdit peptide est préalablement solubilisé dans un ou plusieurs solvants cosmétiquement ou pharmaceutiquement acceptables tel(s) que l'eau, le propylène glycol, le butylène glycol, les diglycols éthoxylés ou propoxylés, l'éthanol, le propanol ou l'isopropanol.

Selon un autre mode de réalisation avantageux de l'invention, ledit peptide est préalablement solubilisé dans un vecteur pharmaceutique comme les liposomes ou adsorbé sur des polymères organiques poudrés, des supports minéraux comme le talc et la bentonite, et plus généralement solubilisé dans, ou fixé sur, tout vecteur pharmaceutiquement acceptable.

Les compositions pharmaceutiques de la présente invention contiennent de 0,00002 à 5 %, de préférence de 0,00005 à 0,1 %, plus préférentiellement encore de 0,0001 à 0,001 % en poids du peptide de l'invention, au moins un excipient pharmaceutiquement acceptable et bien connu(s) de l'homme du métier tel que par exemple des solvants, épaississants, diluants, tensioactifs, anti-oxydants, colorants, conservateurs, et/ou parfums, en fonction de la formulation finale de la composition de l'invention.

Les inventeurs ont pu montrer que des quantités, même très faibles (de l'ordre de 0,00002% en poids du peptide de l'invention dans une composition pharmaceutique ou cosmétique) étaient suffisantes pour obtenir l'activité requise. La présente invention décrit des quantités maximales de 5 % en poids du peptide de l'invention dans une composition pharmaceutique ou cosmétique, pour des raisons économiques. Néanmoins l'homme du métier pourra envisager des quantités supérieures à 5 % et adapter la concentration, si nécessaire à l'obtention d'une action au moins égale, en fonction du coût et de la pureté du peptide selon l'invention.

Plus particulièrement, la composition selon la présente invention comprend en outre au moins un autre principe dermatologiquement actif agissant soit sur le renforcement de la jonction dermo-épidermique, l'adhérence cellules-matrice de la peau, et/ou l'adhérence cellule-cellule soit autrement sur la peau selon la nature de ou des agent(s) utilisé (s) tels qu'un agent hydratant, un agent relipidant, un agent surgraissant, un agent exfoliant, un agent kératolytique, un agent antioxydant, un agent apaisant, un agent adoucissant, un agent sédatif, un agent nettoyant, un agent démaquillant, un agent assainissant, un agent antibactérien, un agent antiseptique, un agent antiséborrhéique, un agent décongestionnant, un agent revitalisant, un agent activateur du renouvellement cellulaire ou un ou plusieurs filtres solaires.

Préférentiellement, cette composition est destinée à une application topique.

Les compositions de l'invention doivent se présenter sous une forme dermatologiquement acceptable, c'est-à-dire compatible avec la peau, les poils et/ou les cheveux. Ces compositions pourront notamment être sous forme de crèmes, laits, émulsions huile dans eau, eau dans huile, émulsions multiples, solutions, suspensions, gels aqueux, gels huileux, gels hydroalcooliques, lotions, sticks ou encore poudres, adaptés à une application sur la peau, les muqueuses et en particulier les lèvres et/ou les cheveux.

D'éventuels composés additionnels, actifs ou non, pourront être ajoutés à la composition de l'invention et l'homme du métier les choisira de façon à ce que leur adjonction n'altère pas les propriétés avantageuses de ladite composition.

L'invention a également pour objet l'utilisation du peptide selon la présente invention pour la préparation d'une composition pharmaceutique destinée à renforcer la jonction dermo-épidermique, l'adhérence cellule-matrice et/ou l'adhérence cellule-cellule au niveau de l'épiderme et favoriser ainsi la réparation de l'épiderme.

Comme indiqué précédemment, l'altération de l'épiderme et de la JDE la plus communément observée est celle résultant du vieillissement cutané. Cependant, d'autres altérations de la peau peuvent intervenir indépendamment du vieillissement et peuvent par exemple être induites par certaines pathologies dermatologiques. A titre d'exemple, on peut citer l'eczéma, le psoriasis, le prurit les dermites irritatives, l'héliodermie, les kératoses, les mycoses, l'ichtyose. Outre les symptômes spécifiques à chacune de ces pathologies, la peau subit des altérations auxquelles se propose de remédier la présente invention à titre de complément thérapeutique. Il est clair que la présente invention n' a pas pour but le traitement de telles pathologies mais de restaurer la JDE endommagée et l'adhérence cellulaire dans ces pathologies.

Ainsi la présente invention permet la réparation, la régénération et/ou la restructuration de la peau.

La peau peut également être fragilisée par des traitements cosmétiques ou thérapeutiques de la peau qui, tout en traitant la pathologie visée, génèrent des effets secondaires sur la peau qu'il convient de traiter indépendamment de la pathologie elle-même. C'est le cas notamment des traitements de l'acné, des traitements par puvathérapie, des interventions chirurgicales (dermatologiques ou autres), des traitements de la peau par laser, de la dermabrasion, des peelings ou des traitements de cancers par radiothérapie.

Enfin, la composition de l'invention est également destinée au traitement curatif et préventif non seulement du vieillissement cutané comme vu plus haut, telles que les rides, le relâchement, la perte d'élasticité et la moins bonne cicatrisation mais également au traitement curatif ou préventif de la xérose sénile, des modifications du système pigmentaire de la peau, de l'appauvrissement du réseau vasculaire de la peau, de l'altération des phanères, de l'irrégularité du grain de peau et de l'atrophie cutanée.

En effet, les susdites pathologies, fragilisations de la peau et traitements divers génèrent sur la peau des altérations telles que la diminution de l'adhérence de l'épiderme et de la cohésion épidermique, ou encore une moins bonne restructuration de la surface cutanée.

L'invention concerne enfin un procédé de traitement cosmétique de la peau caractérisé en ce qu'on applique sur la peau une composition cosmétique comprenant au moins un peptide selon l'invention. Ladite composition cosmétique selon l'invention contient de 0,00002 % à 5 %, de préférence de 0,00005 % à 0,1 % et plus préférentiellement encore de 0,0001 % à 0,001 % en poids de peptide et au moins un excipient cosmétiquement acceptable.

Selon l'invention, ladite composition cosmétique peut également comprendre au moins un autre principe cosmétiquement actif.

Selon la présente invention, ladite composition cosmétique se présente sous la forme d'une crème, d'un lait, d'une émulsion huile dans l'eau, d'une émulsion eau dans l'huile, d'une émulsion multiple, d'une solution, d'une suspension, d'un gel aqueux, d'un gel huileux, d'un gel hydroalcoolique, d'une lotion, d'un stick ou d'une poudre.

D'autres avantages et caractéristiques de l'invention apparaîtront mieux à la lecture des exemples donnés à titre illustratif et non limitatif.

## LEGENDES DES FIGURES:

Figure 1 : Schéma de la chaîne gamma 2 de la laminine 5 (155 kDa).

Figure 2 : Adhérence cellulaire des HBL1000 aux peptides 1, 2 et 3.
Adhérence cellulaire dose dépendante des cellules de la lignée HBL100 aux différents peptides 1, 2 et 3. Les peptides ont été immobilisés sur des plaques à 96 puits aux concentrations indiquées en abscisse. $8.10^4$ cellules ont été déposées dans chaque puits et les plaques ont été incubées à 37°C pendant 1 heure. Après lavages, les cellules adhérées ont été fixées et l'adhérence cellulaire a été mesurée comme décrit dans la partie méthodologie. Les cellules ont été observées en microscopie à contraste de phase puis photographiées. *Barre = 50 μm.*

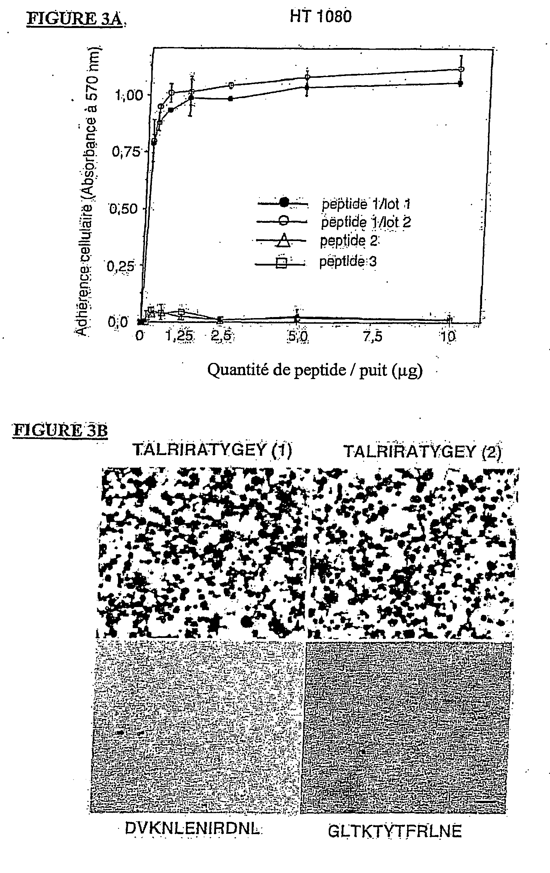

Figure 3 : Adhérence cellulaire des HT1080 aux peptides 1, 2 et 3.

Adhérence cellulaire dose dépendante des cellules de la lignée HT1080 aux différents peptides 1, 2 et 3. Les peptides ont été immobilisés sur des plaques à 96 puits aux concentrations indiquées en abscisse. $8.10^4$ cellules ont été déposées dans chaque puits et les plaques ont été incubées à 37°C pendant 1 heure. Après lavages, les cellules adhérées ont été fixées et l'adhérence cellulaire a été mesurée comme décrit dans la partie méthodologie. Les cellules ont été observées en microscopie à contraste de phase puis photographiées. *Barre = 50 μm.*

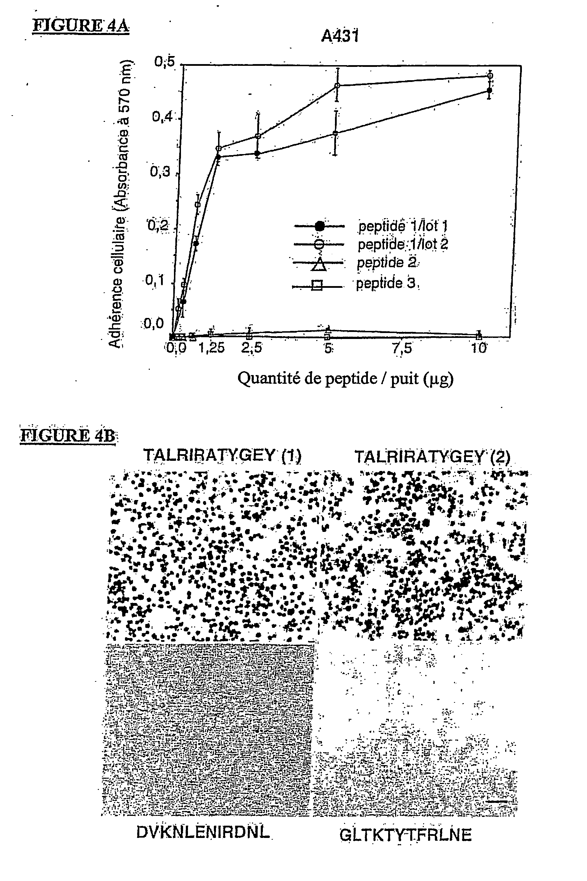

Figure 4 : Adhérence cellulaire des A431 aux peptides 1, 2 et 3.
Adhérence cellulaire dose dépendante des cellules de la lignée A431 aux différents peptides 1, 2 et 3. Les peptides ont été immobilisés sur des plaques à 96 puits aux concentrations indiquées en abscisse. $8.10^4$ cellules ont été déposées dans chaque puits et les plaques ont été incubées à 37°C pendant 1 heure. Après lavages, les cellules adhérées ont été fixées et l'adhérence cellulaire a été mesurée comme décrit dans la partie méthodologie. Les cellules ont été observées en microscopie à contraste de phase puis photographiées. *Barre = 50 μm.*

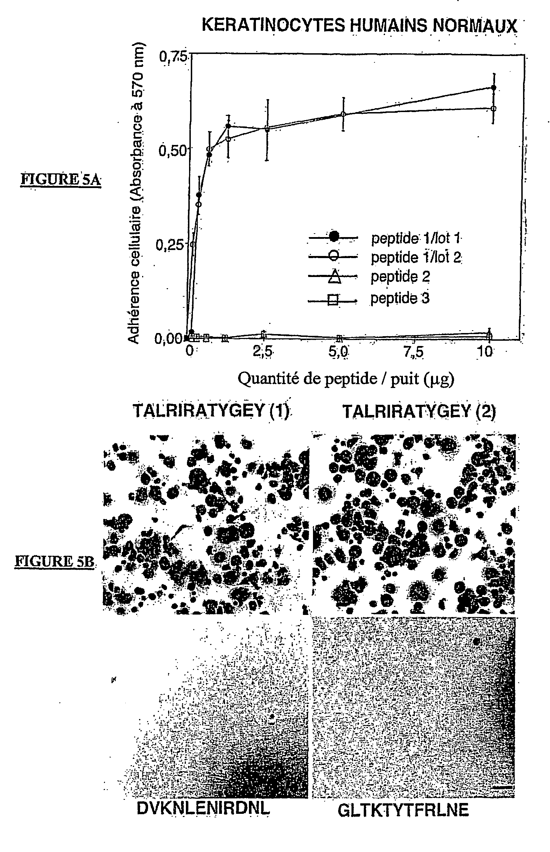

Figure 5 : Adhérence cellulaire des kératinocytes humains normaux aux peptides 1, 2 et 3.
Adhérence cellulaire dose dépendante des kératinocytes humains normaux aux différents peptides 1, 2 et 3. Les peptides ont été immobilisés sur des plaques à 96 puits aux concentrations indiquées en abscisse. $10^5$ cellules ont été déposées dans chaque puits et les plaques ont été incubées à 37°C pendant 1 heure. Après lavages, les cellules adhérées ont été fixées et l'adhérence cellulaire a été mesurée comme décrit dans la partie méthodologie. Les cellules ont été observées en microscopie à contraste de phase puis photographiées. *Barre = 50 μm.*

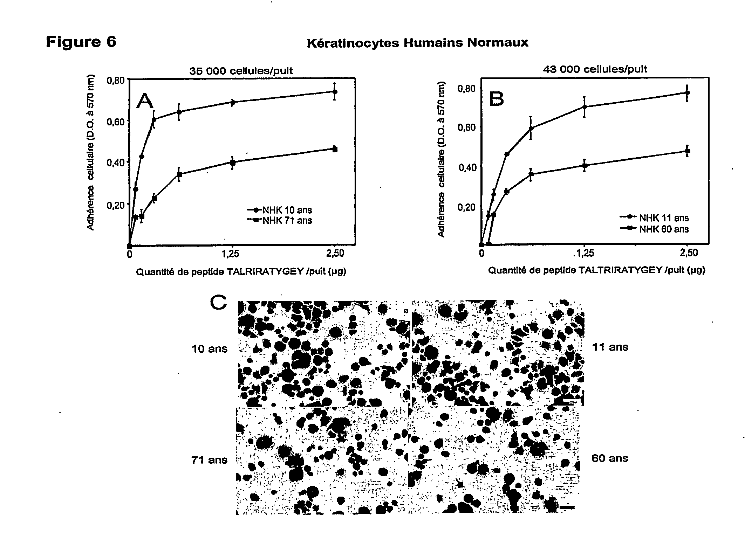

Figure 6 : Adhérence cellulaire des NHK-jeunes versus NHK-âgés au peptide TALRIRATYGEY
Adhérence cellulaire dose dépendante des NHK-10 ans versus NHK-71ans (A) et des NHK-11 ans versus NHK-60 ans (B) au peptide TALRIRATYGEY. Les peptides ont été immobilisés sur des plaques 96 puits aux quantités indiquées en abscisse. $3,5.10^4$ cellules (A) et $4,3.10^4$ cellules (B) ont été déposées dans chaque puit et les plaques ont été incubées à 37 °C pendant 1 heure. Après lavages, les cellules adhérées ont été fixées et l'adhérence cellulaire a été mesurée comme décrit dans la partie méthodologie. (C) Les cellules ont été observées en microscopie à contraste de phase puis photographiées. Barre, 50 μm.

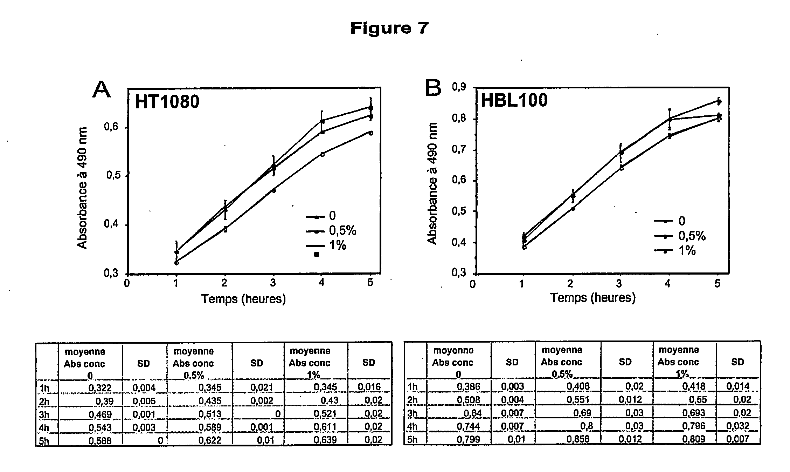

Figure 7 : Effet du peptide TALRIRATYGEY sur la prolifération des cellules HT1080 et HBL100
Les cellules HT1080 (A) et les cellules HBL100 (B) ont été ensemencées dans des plaques 96 puits à raison de 10 000 cellules par puit. Après 24 heures, les milieux de culture ont été enlevés et remplacés par du milieu sans sérum contenant les quantités de peptide indiquées sur les graphes et le réactifXTT. Les plaques ont ensuite été placées dans un incubateur à 37°C et des lectures de l'absorbance ont été effectuées à 1h, 2h, 3h, 4h et 5h. Des témoins sans peptide ont été réalisés sur la même plaque.

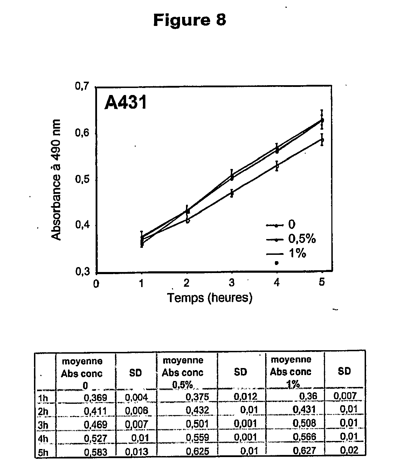

Figure 8 : Effet du peptide TALRIRATYGEY sur la prolifération des cellules A431
Les cellules A431 ont été ensemencées dans des plaques 96 puits à raison de 10 000 cellules par puit. Après 24 heures, les milieux de culture ont été enlevés et remplacés par du milieu sans sérum contenant les quantités de peptide indiquées sur les graphes et le réactifXTT. Les plaques ont ensuite été placées dans un incubateur à 37°C et des lectures de l'absorbance ont été effectuées à 1h, 2h, 3h, 4h et 5h. Des témoins sans peptide ont été réalisés sur la même plaque.

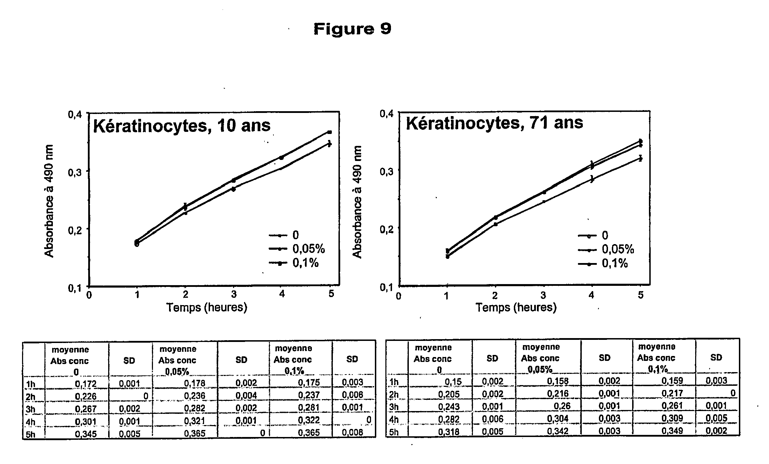

Figure 9 : Effet du peptide TALRIRATYGEY sur la prolifération des NHK-jeunes et des NHK-âgés.
Les NHK-10 ans et NHK-71 ans ont été ensemencés dans des plaques 96 puits à raison de 10 000 cellules par puit. Après 24 heures, les milieux de culture ont été enlevés et remplacés par du milieu sans sérum contenant les quantités de peptide indiquées sur les graphes et le réactif XTT. Les plaques ont ensuite été placées dans un incubateur à 37°C et des lectures de l'absorbance ont été effectuées à 1h, 2h, 3h, 4h et 5h. Des témoins sans peptide ont été réalisés sur la même plaque.

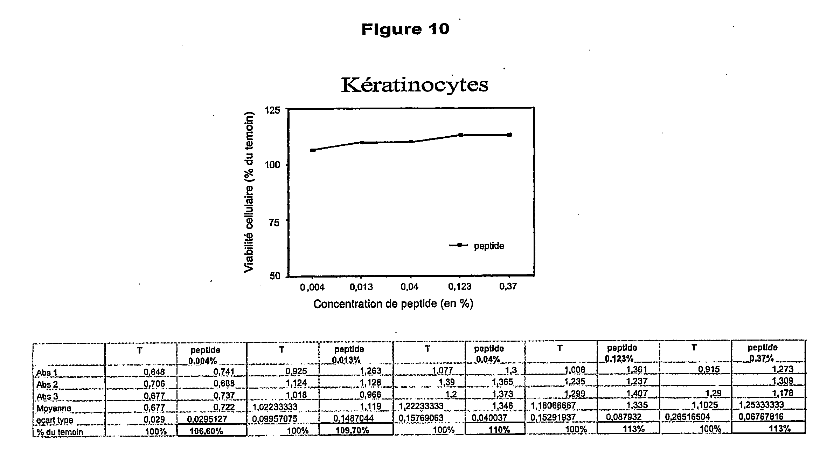

Figure 10 : Effet du peptide TALRIRATYGEY sur la prolifération des NHKs.
Les NHKs ont été ensemencées dans des plaques 96 puits à raison de 10 000 cellules par puit. Après 24 heures, les milieux de culture ont été enlevés et remplacés par du milieu KBM-2 contenant les quantités de peptide indiquées. Après 24h de contact à 37°C, les milieux ont été remplacés par le réactifXTT. Les plaques ont ensuite été placées dans un incubateur à 37°C et la lecture de l'absorbance a été effectuée à 3h. Des témoins sans peptide ont été réalisés sur la même plaque.

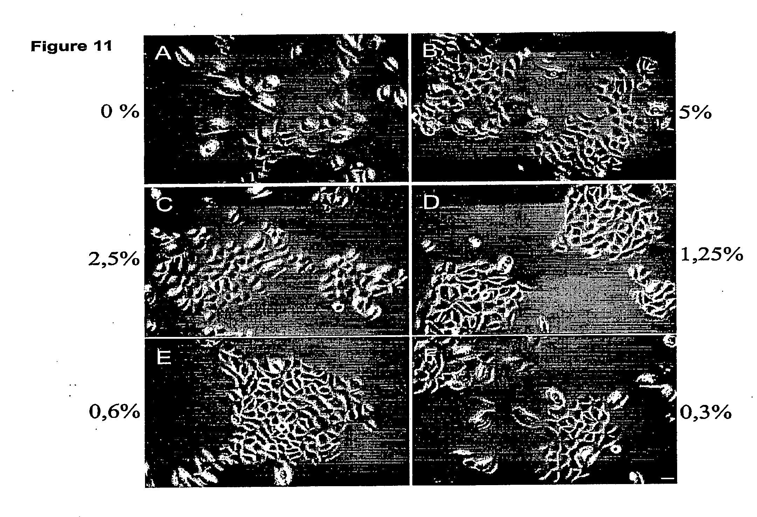

Figure 11 : Effet du peptide TALRIRATYGEY sur le comportement des NHKs.
Les NHKs ont été ensemencées dans des plaques 12 puits à raison de 5 000 cellules par puit. Après 24 heures de culture, les milieux de culture ont été enlevés et remplacés par du milieu neuf contenant les quantités de peptide 5% ; 2,5% ; 1,25% ; 0,6% et 0,3% (B-F). Un témoin sans peptide a été réalisés (A). La plaque a ensuite été placée dans un incubateur à 37°C pendant 48 heures. L'observation a été faite avec un microscope Axiovert 40 Zeiss. Barre, 50 $\mu$m.

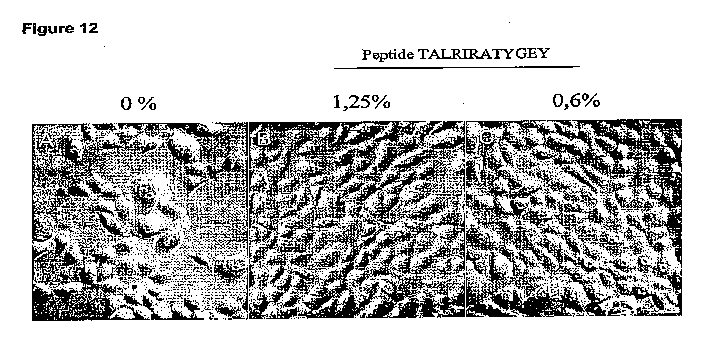

Figure 12 : Effet du peptide TALRIRATYGEY sur le comportement des NHKs.
Observation, à plus fort grandissement, des colonies cellulaires décrites Figure 6. L'observation a été faite avec un microscope Axiovert 40 Zeiss équipé d'un bloc interférentiel PlasDic. Barre, 50 $\mu$m.

Figure 13
(A) Représentation schématique de la laminine 5 et du collagène IV dans la jonction dermo-épidermique. (B) Adhérence cellulaire dose dépendante des NHKs à la laminine 5 et au collagène IV. La laminine 5 et le collagène IV ont été immobilisés sur des plaques 96 puits aux quantités indiquées en abscisse. $5.10^4$ NHKs ont été déposées dans chaque puit et les plaques ont été incubées à 37 °C pendant 1 heure. Après lavages, les cellules adhérées ont été fixées et l'adhérence cellulaire a été mesurée comme décrit dans la partie méthodologie. (C) Les cellules ont été observées en microscopie à contraste de phase puis photographiées. Barre, 50 $\mu$m.

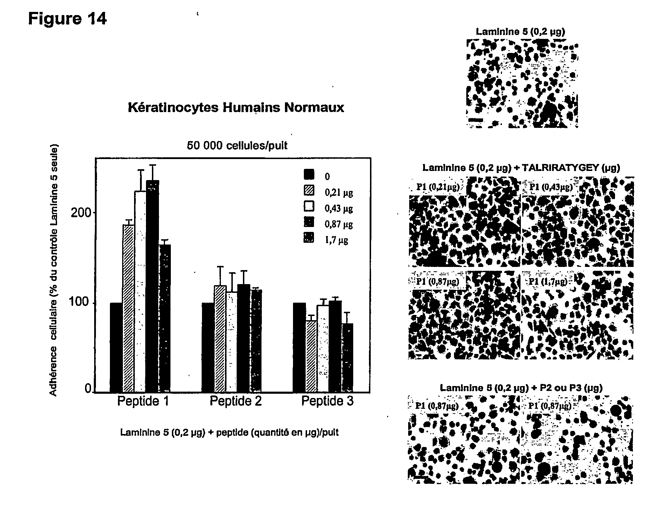

Figure 14: Adhérence cellulaire des NHKs au peptide TALRIRATYGEY co-immobilisé avec la laminine 5.
Les peptides 1, 2 et 3 (quantités variables indiquées) et la laminine 5 (quantité fixe 0,2 mg) ont été co-immobilisés sur des plaques 96 puits. 5.104 NHKs ont été déposées dans chaque puit et les plaques ont été incubées à 37 °C pendant 1 heure. Après lavages, les cellules adhérées ont été fixées et l'adhérence cellulaire a été mesurée comme décrit dans la partie méthodologie. L'adhérence cellulaire en présence du peptide a été présentée en pourcentage de l'adhérence cellulaire obtenue à la laminine 5 seule. Les cellules ont été observées en microscopie à contraste de phase puis photographiées. Barre, 50 $\mu$m.

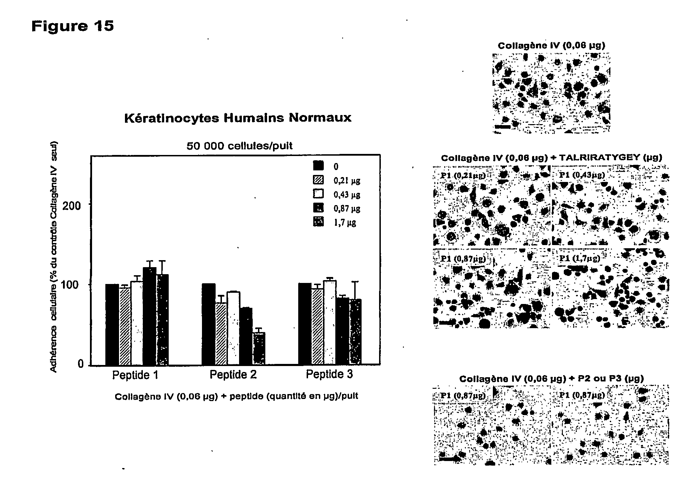

Figure 15: Adhérence cellulaire des NHKs au peptide TALRIRATYGEY co-immobilisé avec le collagène IV.
Les peptides 1,2 et 3 (quantités variables indiquées) et le collagène IV (quantité fixe 0,06 mg) ont été co-immobilisés sur des plaques 96 puits. 5.104 NHKs ont été déposées dans chaque puit et les plaques ont été incubées à 37 °C pendant 1 heure. Après lavages, les cellules adhérées ont été fixées et l'adhérence cellulaire a été mesurée comme décrit dans la partie méthodologie. L'adhérence cellulaire en présence du peptide a été présentée en pourcentage de l'adhérence cellulaire obtenue au collagène IV seul. Les cellules ont été observées en microscopie à contraste de phase puis photographiées. Barre, 50 $\mu$m.

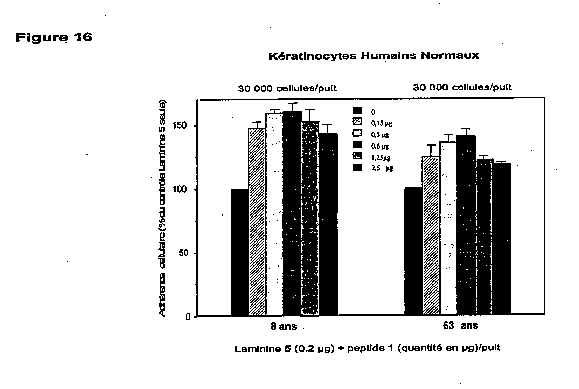

Figure 16 : Adhérence cellulaire des NHK-jeunes versus NHK-âgés au Peptide TALRIRATYGEY co-immobilisé avec la laminine 5.
Le peptide (quantités variables indiquées) et la laminine 5 (quantité fixe 0,2 mg) ont été co-immobilisés sur des plaques 96 puits. 3.104 NHK-8 ans et 3.104 NHK-63 ans ont été déposées dans chaque puit et les plaques ont été incubées à 37 °C pendant 1 heure. Après lavages, les cellules adhérées ont été fixées et l'adhérence cellulaire a été mesurée comme décrit dans la partie méthodologie. L'adhérence cellulaire en présence du peptide a été présentée en pourcentage de l'adhérence cellulaire obtenue à la laminine 5 seule.

Figure 17
Tableau récapitulatif des dosages de peptide TALRIRATYGEY non immobilisé sur les plaques 96 puits.

Figure 18
Tableau récapitulatif des dosages de peptide TALRIRATYGEY non immobilisé sur les plaques 96 puits.

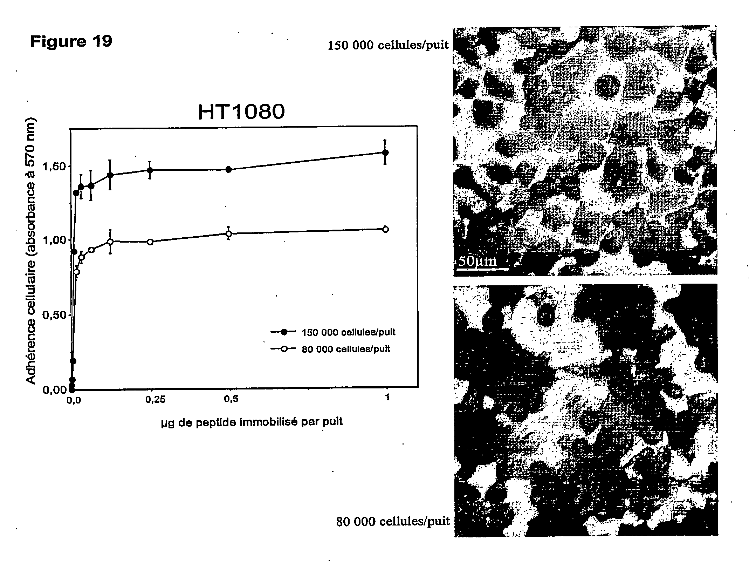

Figure 19: Adhérence des cellules HT1080 au peptide TALRIRATYGEY
Adhérence cellulaire dose dépendante des cellules HT1080 au peptide TALRIRATYGEY. Les peptides ont été immobilisés sur des plaques 96 puits aux quantités indiquées en abscisse. 8.104 cellules et 15.104 cellules ont été déposées dans chaque puit et les plaques ont été incubées à 37 °C pendant 1 heure. Après lavages, les cellules adhérées ont été fixées et l'adhérence cellulaire a été mesurée comme décrit dans la partie méthodologie. Les cellules ont été observées en microscopie à contraste de phase puis photographiées. Barre, 50 $\mu$m.

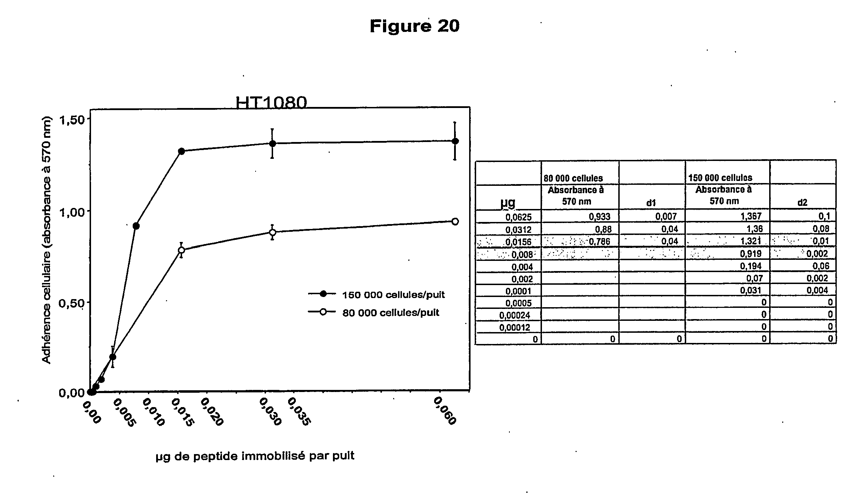

Figure 20: Adhérence des cellules HT1080 au peptide TALRIRATYGEY
Adhérence cellulaire dose dépendante des cellules HT1080 au peptide TALRIRATYGEY. Les peptides ont été immobilisés sur des plaques 96 puits aux quantités indiquées en abscisse. 8.104 cellules et 15.104 cellules ont été déposées dans chaque puit et les plaques ont été incubées à 37 °C pendant 1 heure. Après lavages, les cellules adhérées ont été fixées et l'adhérence cellulaire a été mesurée comme décrit dans la partie méthodologie. Les valeurs des absorbances à 570 nm sont indiquées dans le tableau.

## EXEMPLES

### EXEMPLE 1 :

Des expériences d'adhérence cellulaire ont été effectuées sur 3 peptides avec des cellules communément utilisées pour les études d'adhérence cellulaire comme les HT1080 (fibrosarcome humain), les HBL100 (épithélium mammaire humain), et les A431 (épithélioïdes de peau) afin de prouver la spécificité d'adhérence du peptide de l'invention.

En plus du peptide de l'invention (peptide 1), deux autres peptides (peptides 2 et 3, correspondant chacun à une séquence de la chaîne gamma 2 de la laminine-5 mais toutes deux différentes de la séquence du peptide de l'invention), ont été synthétisés et ont été utilisés comme contrôle lors des expériences d'adhérence cellulaire.

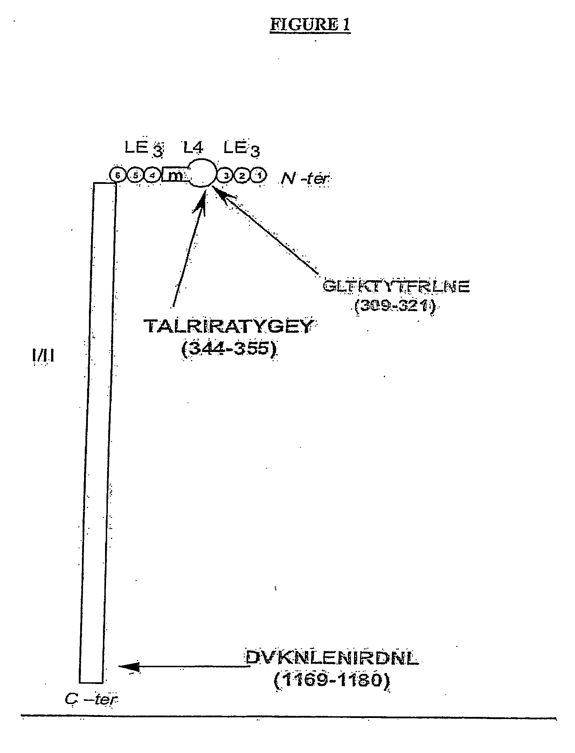

Les peptides 1 et 2 sont situés au niveau de l'extrémité amino-terminale dans la région appelé « bras court » de la chaîne gamma 2 (Figure 1). Le bras court des laminines comporte des séquences pauvres en cystéines repliées en un domaine globulaire (le domaine L4m). Ce domaine est entouré de répétitions riches en cystéines agencées en bâtonnets (les domaines LE, 3 domaines LE de chaque côté). Ces derniers sont composés d'un motif de 50 acides aminés présentant une homologie avec le facteur de croissance épidermique (domaines type EGF). Le peptide 3 a été choisi au niveau de l'extrémité carboxy-terminale. Les domaines caboxy-terminaux I et II sont impliqués dans l'assemblage des trois chaînes alpha, bêta et gamma et forment une hélice alpha superenroulée formant le bras long de la laminine. La séquence d'intérêt est localisée sur le domaine globulaire L4m.

Au cours des expériences d'adhérence cellulaire, des effets doses-réponses ont été effectuées et le phénotype des cellules adhérées analysé en microscopie à contraste de phase (prise de photos).

### I- Informations sur le peptide de l'invention : TALRIRATYGEY (SEQ ID N° 1)

Nombre d'acides amines : 12
Poids moléculaire: 1413,6
Point Isoélectrique théorique : 8,25

Composition en acides aminés :

| Ala (A) | 2 | 16,7% | Met (M) | 0 | 0,0 % |
|---------|---|-------|---------|---|-------|
| Arg(R) | 2 | 16,7% | Phe (F) | 0 | 0,0 % |
| Asn(N) | 0 | 0,0 % | Pro (P) | 0 | 0,0 % |
| Asp (D) | 0 | 0,0 % | Ser (S) | 0 | 0,0 % |
| Cys (C) | 0 | 0,0 % | Thr (T) | 2 | 16,7% |
| Gln(Q) | 0 | 0,0 % | Trp (W) | 0 | 0,0 % |
| Glu (E) | 1 | 8,3% | Tyr (Y) | 2 | 16,7% |
| Gly (G) | 1 | 8,3% | Val (V) | 0 | 0,0 % |
| His(H) | 0 | 0,0 % | Asx (B) | 0 | 0,0 % |
| Ile (I) | 1 | 8,3% | Glx (Z) | 0 | 0,0 % |
| Leu (L) | 1 | 8,3% | Xaa(X) | 0 | 0,0 % |
| Lys(K) | 0 | 0,0 % | | | |

Nombre total de résidus chargés négativement (Asp + Glu) : 1
Nombre total de résidus chargés positivement (Arg + Lys) : 2

Composition atomique :

| | | |
|---|---|---|
| Carbone | C | 63 |
| Hydrogène | H | 100 |
| Azote | N | 18 |
| Oxygène | O | 19 |
| Soufre | S | 0 |

Formule : C63H100N18O19

Nombre total d'atomes : 200

Coefficient d'extinction :

Conditions: 6,0 M d'hydrochlorure de guanidium

0,02 M de tampon phosphate

pH 6,5

Les unités des coefficients d'extinction sont en $M^{-1}$ $cm^{-1}$

| | 276 nm | 278 nm | 279 nm | 280 nm | 282 nm |
|---|---|---|---|---|---|
| Ext. coefficient | 2900 | 2800 | 2690 | 2560 | 2400 |
| Abs 0,1 %(=1 g/l) | 2.051 | 1.981 | 1.903 | 1.811 | 1.698 |

Demi-vie estimée :

Le N-terminal de la séquence considérée est T (Thr).

La demi-vie estimée est : 7,2 heures (réticulocytes de mammifères, *in vitro*).

>20 heures (levures, *in vivo*).

>10 heures (*Escherichia coli, in vivo*).

Index d'instabilité :

L'index d'instabilité a été calculé à 11,86

Ceci classifie la protéine comme stable.

Index aliphatique : 81,67

Moyenne d'hydropathie : - 0,417

**II- Matériels et méthodes**

1°) Procédé de fabrication des peptides

La synthèse peptidique a été effectuée sur un Synthétiseur Milligen 9050 en utilisant une chimie Fmoc-Opfp/ Hobt. Le peptide est ensuite analysé et purifié sur une colonne Vydac C 18 (5 $\mu$m) de 4,6 ou 10 mm de diamètre et 250 mm de longueur puis caractérisé par spectrométrie de masse électrospray sur un SCIEX API 165.

2°) Séquence des peptides

Peptide 1 : TALRIRATYGEY (SEQ ID N° 1)

Position 344-355 (Figure 1)

Peptide 2 : GLTKTYTFRLNE (SEQ ID N° 2)

Position 309-321 (Figure 1)
Peptide 3 : DVKNLENIRDNL (SEQ ID N° 3)
Position 1169- 1180(Figure 1)

3) <u>Analyste quantitative des propriétés d'adhérence cellulaire du peptide de l'invention par un test colorimétrique</u>

- <u>Préparation des substrats d'adhérence</u>

Les peptides 1, 2 et 3 ont été utilisés au cours des expériences d'adhérence cellulaire. Une gamme de 7 concentrations décroissantes (100 microgrammes/ml; 50 microgrammes/ml; 25 microgrammes/ml; 12,5 microgrammes/ml; 6,25 microgrammes/ml; 3,125 microgrammes/ml et 1,562 microgrammes/ml) a été réalisée par dilution successive dans du PBS (Phosphate Buffer Saline, $KH_2PO_4$ 1,54 mM ; $Na_2HPO_4$ 1,42 mM ; NaCl 131 mM), à partir d'une solution de départ à 1 mg/ml. Ces solutions ont été immédiatement distribuées sur des plaques de culture à 96 puits (Greinher, Dutscher, Brumath, France) à raison de 100 $\mu$l par puits. Les plaques ont ensuite été placées à + 4°C pendant 16 à 18 heures. Les solutions ont ensuite été enlevées par retournement des plaques et chaque puits a été saturé par une solution de PBS-SAB 1% (sérum albumine bovine). Trois puits supplémentaires sans substrat ont subi le même traitement et ont servi de blanc.

- <u>Test d'adhérence cellulaire</u>

Les cellules épithéliales ont été détachées des boîtes de culture par une solution de trypsine/EDTA (0,05-0,02 %), puis ont été suspendues dans du milieu DMEM sans additifs pour les lignées cellulaire et KBM-2 sans additif pour les kératinocytes humains. Le nombre de cellules semées par puits est indiqué dans les légendes des graphes (50 000 à 100 000 cellules par puits).

- <u>Evaluation du test d'adhérence cellulaire</u>

Après ensemencement des cellules, les plaques multipuits ont été placées dans un incubateur à 37°C sous atmosphère de CO2 à 5 %. Après une incubation de 30 à 60 minutes, les cellules sont observées au microscope à contraste de phase afin de vérifier que le test s'est correctement déroulé. Le milieu d'adhérence est alors éliminé et chaque puits est lavé avec une solution de PBS stérile afin d'enlever les cellules n'ayant pas adhérées. Les cellules restantes, adhérentes au substrat, sont ensuite fixées à l'aide d'une solution de glutaraldéhyde à 1 % dans du PBS, pendant 15 minutes. La solution de glutaraldéhyde est enlevée et les cellules sont alors colorées avec une solution de crystal violet dilué à 1 % dans de l'eau distillée pendant 30 minutes.

Après plusieurs rinçages à l'eau, les cellules sont perméabilisées par une solution de triton à 0,02 % pendant 15 minutes, afin de solubiliser le crystal violet. La lecture de l'absorbance est effectuée à 570 nm, à l'aide d'un lecteur de plaques ELISA reader (MR500, Dynatech, Guernsey Chanel, Island). Chaque point expérimental a été réalisé en trois exemplaires. La valeur du blanc représente la moyenne de l'absorbance des 3 puits témoins (SAB). Celle-ci a été soustraite à chacune des valeurs de densité optique obtenues pour les points expérimentaux. On a ensuite calculé les moyennes des trois valeurs d'absorbance pour chacune des tripliquettes.

Les résultats ont été représentés sous forme de courbe, avec en ordonnée, les valeurs de l'absorbance, et en abscisse, les différentes concentrations en substrat. Les cellules adhérées ont été photographiées en microscopie à contraste de phase.

4) <u>Les cellules utilisées pour l'étude</u>

*A- les lignées*

Dans un premier temps les cellules suivantes provenant de lignées épithéliales (couramment utilisées pour les études préliminaires d'adhérence cellulaire) ont été utilisées :

- les cellules de la lignée HT1080 (fibrosarcome humain), American Type Culture Collection CCL-121.
- les cellules de la lignée HBL100 (épithélium mammaire humain), American Type Culture Collection HTB -124.
- les cellules de la lignée A431 (épithélioïdes de peau), American Type Culture Collection CRL-1555.

Ces cellules ont été maintenues en culture dans du milieu DMEM supplémenté avec 10% de sérum de veau foetal et 2mM de glutamine et ont été cultivés à 37°C dans un incubateur à CO2 (5 % de CO2, 95 % d'air et 98 % d'humidité).

*B- les kératinocytes primaires*

Dans un deuxième temps des kératinocytes humains normaux fraîchement isolés ont été utilisés. En effet, les kératinocytes basaux de l'épiderme étant en contact direct avec la LN-5 dans la peau, il était nécessaire de tester leur capacité d'adhérence sur le peptide d'intérêt. Les kératinocytes humains ont été obtenus à partir de biopsie de prépuce (déchet opératoire, Pavillon T-Bis, Hôpital Edouard Herriot). Le milieu de culture utilisé au cours de notre travail a été le milieu défini pour culture de kératinocytes KBM-2 (contenant : Extrait pituitaire bovin 35 mg, hEGF 10 ng/ml, insuline 5 $\mu$g/ml, Hydrocortisone 0,5 $\mu$g/ml, transferrine 0,1 %, épinéphrine 0,1 %) fabriqué par Clonetics et commercialisé par Cambrex (Belgique) contenant 0,15 mM de CaCl2, pH 7,2 à 7,4.

Les kératinocytes ont été obtenus selon la technique décrite par Boyce et Ham (Cultivation, frozen storage, and clonal growth of normal human epidermal keratinocytes in serum free-media, Tiss Cult. Meth. 1985,9 :83-93). Les morceaux de peau, après rinçage soigné dans du tampon PBS contenant des antibiotiques, sont débarrassés du tissu graisseux situé sous le derme à l'aide d'instruments stériles. La peau a ensuite été découpée en morceaux de 3 mm$^2$, lesquels ont été placés dans une solution stérile de trypsine à 0,25 % dans du PBS pendant 16 heures à 4°C. La séparation derme / épiderme a été effectuée à l'aide de pinces fines dans une boîte de Pétri contenant du milieu de culture afin d'arrêter l'action enzymatique de la trypsine. Les fragments d'épiderme ont été aspirés et refoulés plusieurs fois avec une pipette pour en détacher les cellules basales libres. La suspension cellulaire ainsi obtenue a été centrifugée 5 min à 1000 tours/min et le culot ainsi obtenu a été suspendu dans un volume connu de KBM-2 pour effectuer un comptage des cellules vivantes à l'aide d'un colorant d'exclusion : le bleu trypan. 3.10$^4$ cellules vivantes ont été ensemencées par cm$^2$ sur des boîtes pour culture de tissus de 25 cm$^2$ (Corning, Polylabo, France). Les kératinocytes ont été cultivés à 37°C dans un incubateur à CO2 (5 % de CO2, 95 % d'air et 98 % d'humidité). Le milieu a été changé tous les deux jours. La sous-culture a eu lieu quand les cellules ont atteint la sous-confluence. Le tapis cellulaire a alors été rincé avec du PBS, puis recouvert d'une solution de Trypsine-EDTA (0,05-0,02 %). Après une courte incubation à 37°C, les cellules se sont détachées du support plastique. Les cellules ont alors été ensemencées dans des boîtes de culture de 75 cm$^2$. La congélation des cellules (3 à 5 millions par ampoule) a été réalisée dans le milieu de culture utilisé, en présence de 10 % de Diméthyl sulfoxyde (DMSO) et 20 % de sérum de veau sous un volume de 1 ml.

## III- Résultats

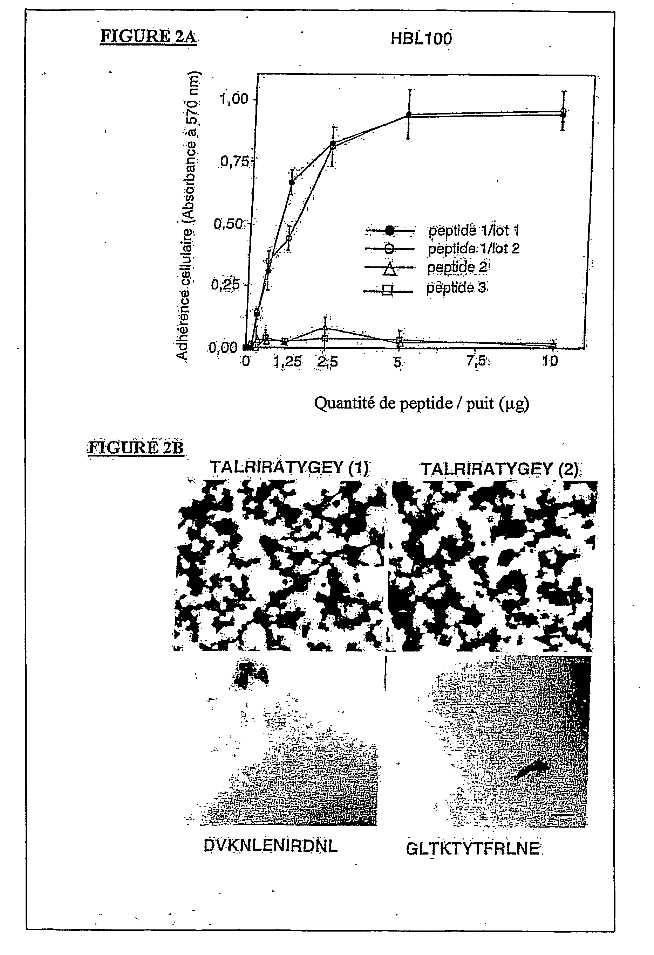

Les expériences d'adhérence cellulaire présentées sur les figures 1 à 5 ont été effectuées avec les quantités de peptides immobilisées suivantes : 0 ; 0,15 ; 0,31 ; 0,62 ; 1,25 ; 2,5 ; 5 ; et 10 microgrammes. Deux lots différents du peptide 1 ont été testés en parallèle. Les peptides 2 et 3 sont des peptides contrôle ; le peptide 2 a été volontairement choisi dans une région proche du peptide 1 dans le domaine L4 de la chaîne gamma 2 et le peptide 3 dans une région éloignée. Le peptide 1 (peptide de l'invention) induit l'adhérence cellulaire des différentes lignées épithéliales testées : HBL100 (Figure 2), HT1080 (Figure 3) et A431 (Figure 4) de façon croissante en fonction de la quantité de peptide immobilisé variant de 0 à 10 microgrammes. Cet effet est également obtenu avec les cellules de l'épiderme : les kératinocytes humains normaux (Figure 5). Les cellules sont solidement ancrées sur le peptide puisqu'elles ont résisté aux différents lavages avant leur fixation. Les deux autres peptides (peptides 2 et 3), choisis au hasard sur la même protéine, n'induisent aucune adhérence quelque soit la quantité immobilisée. L'adhérence des kératinocytes humains normaux est induite pour une quantité de peptide 1 immobilisée de 0,15 microgrammes et l'adhérence maximale est atteinte pour une quantité de peptide 1 immobilisée de 0,62 microgrammes (Figure 5). L'adhérence des cellules HBL 100 est induite pour une quantité de peptide 1 immobilisée de 0,31 microgrammes et l'adhérence maximale est atteinte pour une quantité de peptide 1 immobilisée de 1,25 microgrammes (Figure 2). L'adhérence des cellules HT1080 est induite pour une quantité de peptide 1 immobilisée de 0,15 microgrammes et l'adhérence maximale est atteinte pour une quantité de peptide 1 immobilisée de 0,15 microgrammes (figure 3). L'adhérence des cellules A431 est induite pour une quantité de peptide 1 immobilisée de 0,62 microgrammes et l'adhérence maximale est atteinte pour une quantité de peptide 1 immobilisée de 1,2 microgrammes.

Les photos obtenues en microscopie à contraste de phase confirment les résultats quantitatifs et confirment l'absence de cellules adhérées sur les peptides contrôles 2 et 3. Les cellules adhérées au peptide TALRIRATYGEY (peptide de l'invention) présentent majoritairement une morphologie arrondie avec des regroupements cellulaires fréquents (Figures 2, 3, 4 et 5). Ces regroupements peuvent être le signe de la mise en place d'interactions cellules-cellules. On note également la présence de prolongements cellulaires qui sont les plus visibles dans le cas des HBL100 (Figure2), des HT1080 (Figure 3) et des kératinocytes humains normaux (Figure 5). On les voit plus difficilement dans le cas des A431, car les cellules sont plus petites. Ces prolongements cellulaires peuvent être le signe d'un réarrangement du cytosquelette de la cellule en réponse à l'adhérence sur le peptide. Les résultats ont été confirmés avec les kératinocytes humains normaux (cellules de l'épiderme, Figure 5).

## EXEMPLE 2

**I. Matériel et méthodes**

1°) Culture des cellules

    a°) Les lignées et kératinocytes humains normaux ( voir Exemple 1)
    b°) Obtention et culture des kératinocytes jeunes et âgés

Pour obtenir des kératinocytes provenant de biopsies de sujets très jeunes et de sujets âgés, prélevés au même site anatomique, les inventeurs ont effectué l'étude sur des biopsies cutanées de la face. La zone du visage située derrière les oreilles a été choisie afin de n'étudier que le vieillissement intrinsèque et de s'affranchir des effets provoqués par le vieillissement photo-induit. Des déchets opératoires provenant d'opérations de chirurgie esthétique (lifting en l'occurrence) chez des sujets d'âge avancé et des déchets opératoires provenant des interventions sur oreilles décollées chez des jeunes enfants ont été collectés. Il était primordial de disposer des biopsies le jour même de l'intervention afin de mettre en culture rapidement, les kératinocytes de l'épiderme. Pour cette étude les cellules de biopsies de sujets âgés (60, 63 et 71 ans) et de sujets jeunes (8,10 et 11 ans) ont été utilisées. Les kératinocytes ont été obtenus selon la technique décrite par Boyce et Ham, 1985. Les morceaux de peau, après rinçage soigné dans du tampon PBS contenant des antibiotiques, ont été débarrassés du tissu graisseux situé sous le derme à l'aide d'instruments stériles. La peau a ensuite été découpée en morceaux de 3 mm$^2$, lesquels ont été placés dans une solution stérile de trypsine à 0,25 % dans du PBS pendant 16 heures à 4°C. La séparation derme / épiderme a été effectuée à l'aide de pinces fines dans une boîte de Pétri contenant du milieu de culture afin d'arrêter l'action enzymatique de la trypsine. Les fragments d'épiderme ont été aspirés et refoulés plusieurs fois avec une pipette pour en détacher les cellules basales libres. La suspension cellulaire ainsi obtenue a été centrifugée 5 min à 1000 tours/min et le culot ainsi obtenu a été suspendu dans un volume connu de milieu KBM-2 pour effectuer un comptage des cellules vivantes à l'aide d'un colorant d'exclusion : le bleu trypan. $3.10^4$ cellules vivantes ont été ensemencées par cm$^2$ sur des boîtes pour culture de tissus de 25 cm$^2$ (Corning, Polylabo, France). Les kératinocytes ont été cultivés à 37°C dans un incubateur à CO2 (5 % de CO2, 95% d'air et 98% d'humidité). Le milieu a été changé tous les deux jours. La sub-culture a eu lieu quand les cellules ont atteint la sub-confluence. Le tapis cellulaire a alors été rincé avec du PBS, puis recouvert d'une solution de Trypsine-EDTA (0,05-0,02 %). Après une courte incubation à 37°C, les cellules se sont détachées du support plastique. Les cellules ont alors été ensemencées dans des boîtes de culture de 75 cm$^2$. La congélation des cellules (3 à 5 millions par ampoule) a été réalisée dans le milieu de culture utilisé, en présence de 10 % de Diméthyl Sulfoxyde (DMSO) et 20 % de sérum de veau sous un volume de 1 ml. Lors de la décongélation, les cellules ont été semées à raison de 10 000 cellules/cm$^2$.

2°) Tests d'adhérence cellulaire

Dose-réponses d'adhérence cellulaire des cellules jeunes et âgées sur le peptide TALRIRATYGEY

Une gamme de 7 quantités décroissantes de peptide TALRIRATYGEY a été réalisée par dilution successive dans du PBS (Phosphate Buffer Saline, KH2PO4 1,54 mM ; Na2HPO4 1,42 mM ; NaCl 131 mN), àpartir d'une solution du peptide à 1 mg/ml. Ces solutions ont été immédiatement distribuées sur des plaques de culture 96 puits (Greiner, Dutscher, Brumath, France) à raison de 100 μl par puit. Les plaques ont été placées à +4°C pendant 16 à 18 heures. Les solutions ont ensuite été enlevées par retournement des plaques et chaque puit a été saturé par une solution de PBS-SAB 1% (sérum albumine bovine). Trois puits supplémentaires sans substrat ont subit le même traitement et ont servi de blanc.

Les kératinocytes provenant de sujets jeunes ou âgés ont été détachées des boîtes de culture par une solution de trypsine/EDTA (0,05-0,02%), puis ont été suspendues dans du milieu KBM-2. Le nombre de cellules semées par puit est indiqué sur les graphes. Le même nombre de cellules jeunes/âgés est utilisé au cours des expériences comparatives.

Après ensemencement des cellules, les plaques multipuits ont été placées dans un incubateur à 37°C sous atmosphère 5 % CO2. Après une incubation de 30 à 60 minutes, les cellules sont observées à l'aide d'un microscope à contraste de phase afin de vérifier que le test se soit correctement déroulé. Le milieu d'adhérence a alors été éliminé et chaque puit a été lavé avec une solution de PBS stérile afin d'enlever les cellules n'ayant pas adhérées. Les cellules restantes, adhérentes au substrat, ont été fixées à l'aide d'une solution de glutaraldéhyde 1 % dans du PBS, pendant 15 minutes. La solution de glutaraldéhyde a été enlevée et les cellules ont été colorées avec une solution de Crystal Violet diluée à 1 % dans de l'eau distillée pendant 30 minutes. Après d'importants rinçages à l'eau, les cellules ont été perméabilisées par une solution de triton à 0,02 % pendant 15 minutes, afin de solubiliser le Crystal Violet. La lecture de l'absorbance a été effectuée à 570 nm, à l'aide d'un lecteur de plaques ELISA-Reader (MR500, Dynatech, Guernsey Chanel, Island). Chaque point expérimental a été réalisé en trois exemplaires. La valeur du blanc représente la moyenne de l'absorbance des 3 puits témoins (SAB). Celle-ci a été soustraite à chacune des valeurs de densité optique obtenues pour les points expérimentaux. On a ensuite calculé les moyennes des trois valeurs d'absorbances pour chacun des triplicatas.

Les résultats ont été représentés sous forme de courbe, avec en ordonnée, les valeurs de l'absorbance, et en abscisse, les différentes quantités de substrat déposées dans les puits. Les cellules adhérées ont été photographiées avec un microscope à contraste de phase.

## II. Résultats et Discussion

Adhérence cellulaire des NHK-jeunes et NHK-âgés

Sachant que le vieillissement cutané est caractérisé par une déficience des interactions cellules-matrice extracellulaire, il était nécessaire de vérifier la capacité des kératinocytes provenant de sujets âgés à pouvoir adhérer au peptide d'intérêt. Deux expériences comparatives d'adhérence cellulaire « NHK-jeunes versus NHK âgés » au peptide TALRIRATYGEY ont été effectuées (Figure 6). Les expériences ont été réalisées avec les quantités de peptides : 0, 0,08 ; 0,15 ; 0,30 ; 0,6 ; 1,25 ; 2,50 et 5 microgrammes déposés par puit. Pour chacune de ces deux expériences, le nombre de NHK-jeunes et NHK-âgés a été identique afin de pouvoir comparer le résultat obtenu. Une expérience a été effectuée avec des kératinocytes provenant d'une biopsie cutanée d'un enfant de 10 ans et de ceux d'un adulte de 71 ans (figure 6A), l'autre expérience a été effectuée avec des kératinocytes provenant d'une biopsie cutanée d'un enfant de 11 ans et de ceux d'un adulte de 60 ans (figure 6B). Le peptide TALRIRATYGEY induit l'adhésion cellulaire des NHK-jeunes et des NHK-âgés de façon dose dépendante. On constate, dans les deux expériences, qu'un plus grand nombre de NHK-jeunes (approximativement 2 fois plus) ont adhéré par rapport aux NHK-âgés. Ce résultat indique que le récepteur cellulaire reconnaissant le peptide TALRIRATYGEY pourrait diminuer avec l'âge. Le point intéressant est qu'il est toujours exprimé de façon significative et qu'il est fonctionnel puisque l'effet dose-réponse du peptide vis-à-vis des NHK-âgés est identique à l'effet dose obtenu avec les NHK-jeunes. Les photos obtenues en microscopie à contraste de phase confirment les résultats quantitatifs. Les NHK-jeunes adhérés au peptide TALRIRATYGEY présentent majoritairement une morphologie arrondie avec des regroupements cellulaires fréquents (comme décrit dans l'exemple 1), on note moins de cellules dans le cas des NHK-âgés. L'adhérence des NHK-jeunes est induite pour la quantité de peptide déposé 0,08 $\mu$g et celle des NHK-âgés pour une quantité déposée variant de 0,08 à 0,15 $\mu$g. Le plateau d'adhérence est atteint dans la fourchette de 0,3 à 0,6 micro grammes de peptide déposé par puit.

## EXEMPLE 3

### I. Matériel et méthodes

1°) Culture des cellules

a°) Les lignées et kératinocytes humains normaux ( voir Exemple 1)
b°) Obtention et culture des kératinocytes jeunes et âgés (voir exemple 2)

2°) Test de prolifération cellulaire

L'effet du peptide sur la prolifération cellulaire a été analysé à l'aide d'un test colorimétrique (Cell Prolifération Kit XTT, Roche Diagnostics, Meylan, France) sur les cellules utilisées pour les tests d'adhérence cellulaire, à savoir : les cellules HT1080, A431, HBL 100 et les kératinocytes humains normaux provenant de sujets jeunes et de sujets âgés.

La réaction chimique du test est basée sur la production de NADPH des cellules vivantes permettant la réduction des sels de tétrazolium XTT jaunes en sels de formazan orange. La mesure de l'absorbance est effectuée à 490 nm à l'aide d'un lecteur de plaques ELISA-Reader. Les cellules ont été ensemencées dans des plaques 96 puits à raison de 10 000 cellules par puit (6 puits/condition) dans du milieu de culture KBM-2. Après 24 heures de culture à 37°C en présence de 5% de $CO_2$, les milieux de culture ont été enlevés et remplacés par du milieu sans sérum contenant les quantités de peptide indiquées sur les graphes et le réactif du test. Les plaques ont ensuite été placées dans un incubateur à 37°C et des lectures de l'absorbance ont été effectuées à 1h, 2h, 3h, 4h et 5h. Des témoins sans peptide ont été réalisés sur la même plaque. Les résultats sont présentés sous 2 formes:

- sous la forme de courbes montrant l'évolution de l'absorbance en fonction du temps
- ou sous la forme du pourcentage de la viabilité des cellules mises en présence du peptide par rapport aux témoins sans peptide. Dans ce cas, la viabilité cellulaire a été calculée selon la formule :

$$\%viabilité = (\text{Abs. cellules avec peptide} / \text{Abs. cellules témoins}) \times 100.$$

3°) Etude de l'effet du peptide apporté sous forme soluble sur le comportement des kératinocytes

Les kératinocytes ont été ensemencés dans des plaques 12 puits (Costar) à raison de 5 000 Cellules / puit dans du milieu KBM-2. Après 24 heures de culture à 37°C en présence de 5% de $CO_2$, les milieux de culture ont été enlevés et remplacés par du milieu KBM-2 contenant les quantités de peptide indiquées sur les Figures (un contrôle sans peptide a été effectué). Après 24 heures de culture, la même quantité de peptide a été ajoutée au milieu de culture et l'expérience à été arrêtée après 24 heures supplémentaires de culture. L'analyse microscopique a été effectuée sans fixation préalable avec un microscope Axiovert 40 Zeiss couplé à une caméra Coolsnap (Roper Scientific, Evry, France).

**II. Résultats et Discussion**

1- Effet du peptide sur la prolifération cellulaire

Dans le but d'analyser l'effet du peptide TALRIRATYGEY sur la prolifération cellulaire les inventeurs ont dans un premier temps analysé l'effet de 2 concentrations différentes 0,5 et 1% sur les cellules HT1080, HBL100 et A431 (Figures 7, 8 et 9). Les cellules ont été ensemencées dans des plaques 96 puits à raison de 10 000 cellules par puit. Après 24 heures de culture, les milieux de culture ont été enlevés et remplacés par du milieu sans sérum contenant les quantités de peptide indiquées sur les graphes et le réactif du test. Les plaques ont ensuite été placées dans un incubateur à 37°C et des lectures de l'absorbance ont été effectuées à 1 h, 2h, 3h, 4h et 5h. Des témoins sans peptide ont été réalisés sur la même plaque. Une augmentation modérée mais régulière de la prolifération des cellules de la lignée HT1080 (Figure 7A), de la lignée HBL1 00 (figure 7B), et de la lignée A 431 (Figure 8) a été observée au cours du temps pour atteindre à la 5ème heure une augmentation moyenne de 8 % par rapport au contrôle. Des temps plus longs ont été testés et n'ont pas montré d'effet plus prononcé. Aucune différence n'a été notée entre les deux concentrations de peptide testées quel que soit le type cellulaire testé. Le test a ensuite été réalisé avec des NHK-jeunes et NHK-âgés (Figure 9). Les quantités de peptide ajoutées ont été réduites (0,05 % et 0,1%) dans le but de réaliser le test dans une gamme de concentrations plus proche des conditions d'utilisation du peptide au cours des tests d'adhérence. A nouveau, une augmentation modérée et régulière de la prolifération a été observée pour atteindre à la 5ème heure une augmentation moyenne n'excédant pas 7 % par rapport au contrôle. Aucune différence significative n'a été observée, ni entre les deux concentrations utilisées, ni en fonction de l'âge des cellules. Une dernière série d'expériences a été effectuée avec les NHKs sur une gamme plus large de concentrations du peptide et sur un temps plus long (24h) (Figure 10). Les résultats sont présentés sous la forme du pourcentage de la viabilité des cellules mises en présence du peptide pendant 24h par rapport aux témoins sans peptide. Dans cette expérience, on note une augmentation de la prolifération cellulaire variant de 6 à 13 % en fonction de la concentration du peptide ajouté (0,004% à 0,37 %).

2- Etude de l'effet du peptide apporté sous forme soluble sur le comportement des kératinocytes en culture

Le peptide TALRIRATYGEY induit l'adhérence cellulaire lorsqu'il est fixé sur les plaques 96 puits (Exemples 1 et 2). Nous avons voulu savoir s'il était capable d'agir sur le comportement des kératinocytes lorsqu'il est ajouté dans le milieu de culture. Pour cela, les NHKs ont été ensemencées dans des plaques 12 puits à raison de 5 000 cellules par puit. Après 24 heures de culture, les milieux de culture ont été enlevés et remplacés par du milieu neuf contenant les quantités de peptide 5% ; 2,5% ; 1,25% ; 0,6% et 0,3%. Un témoin sans peptide a été réalisé dans la même plaque. La plaque a ensuite été placée dans un incubateur à 37°C et l'opération a été répétée une fois pour 24 heures de culture supplémentaires. Dans le puit témoin (sans ajout de peptide), les NHKs sont, pour la majorité, encore isolés et commence à se rassembler en colonie (Figure 11A). On constate que les NHKs cultivés en présence du peptide sont déjà rassemblés en colonies de plus grande taille bien identifiables (Figure 11B-F). L'observation de ces colonies à plus fort grossissement (Figure 12), montre que les cellules cultivées en présence de peptide forment un tapis cellulaire jointif et cohésif. On n'observe pas de différence morphologique entre les cellules cultivées en présence du peptide (Figure 12B,C) et les cellules contrôles (Figure 12A). Cependant l'ajout du peptide dans le milieu des NHKs a permis la formation de colonies cohésives plus rapidement. Cet effet peut être la conséquence de la petite augmentation de la prolifération cellulaire induite par le peptide.

EXEMPLE 4

**I. Matériel et méthodes**

1°) Culture des cellules

   a°) Les lignées et kératinocytes humains normaux ( voir Exemple 1)
   b°) Obtention et culture des kératinocytes jeunes et âgés (voir exemple 2)

2°) Test d'adhérence cellulaire

Analyse de l'effet du peptide d'intérêt sur l'adhérence des kératinocytes à la laminine 5 (LN5) et au collagène IV (COL4)

Une gamme de quantités décroissantes de LN5 (préparée au laboratoire) et de COL4 (BD Biosciences, Le Pont de Claix, France) ont été réalisée par dilution successive dans du PBS. Ces solutions ont été immédiatement distribuées dans des plaques de culture 96 puits (Costar) à raison de 100 µl par puit. Le déroulement de l'expérience est identique à ce qui est décrit dans l'exemple 2). Les résultats ont été représentés sous forme de courbe, avec en ordonnée, les valeurs de l'absorbance représentative de l'adhérence, et en abscisse, les différentes quantités de substrat déposées dans les puits. Les cellules adhérées ont été photographiées avec un microscope à contraste de phase.

**II. Résultats et Discussion**

Analyse de l'effet du peptide sur l'adhésion des kératinocytes à la Laminine 5 et au collagène IV.

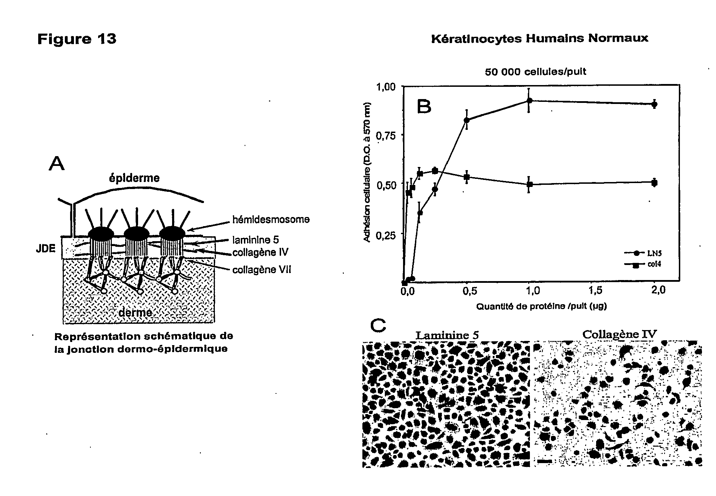

Le réseau moléculaire de base de la jonction dermo-épidermique (JDE) est constitué par l'assemblage des molécules de collagène IV (COL4). Ce réseau moléculaire conduit à la formation d'un filet à mailles lâches, structure polygonale servant de charpente pour arrimer les autres protéines de la lame basale (Figure 13A). Un second réseau moléculaire, est formé par les molécules de laminine. La laminine 5 (LN5) est la glycoprotéine quantitativement la plus importante de la jonction dermo-épidermique, elle est le composant des filaments d'ancrage. La LN5 joue un rôle crucial et irremplaçable dans l'adhérence de l'épiderme par le biais d'interactions avec les intégrines. Dans le but d'analyser l'effet du peptide TALRIRATYGEY dans le contexte de la JDE, les inventeurs ont effectué des expériences de co-immobilisation du peptide avec la LN5 et le COL4 et analysé les capacités d'adhérence des kératinocytes. Dans un premier temps, pour définir les conditions expérimentales, des expériences d'adhérence aux substrats (LN5 et COL4) seuls ont été effectuées. Les expériences ont été réalisées avec les quantités de peptides : 0 ; 0,03 ; 0,06 ; 0,125 ; 0,25 ; 0,5 ; 1 et 2 microgrammes par puit. Comme largement décrit dans la littérature et illustré sur la figure 13B, les 2 protéines testées induisent l'adhérence des NHKs mais la LN5 est un bien meilleur substrat. Les photographies obtenues en microscopie à contraste de phase confirment les résultats quantitatifs et montrent que la LN5 induit la formation d'un tapis cellulaire épithélial cohésif alors et le COL4 induit une adhérence plus faible (Figure 13 C). Pour la suite des expériences, une quantité de substrat (LN5 et COL4) inductrice d'une adhérence moyenne ont été sélectionnées sur les courbes présentées Figure 13B : 0,2 microgramme de LN5 et 0,03 microgramme de COL4.
Pour analyser l'effet du peptide sur l'adhérence cellulaire aux protéines de la JDE, le peptide TALRIRATYGEY (peptide 1) a été co-immobilisé avec la LN5 (Figure 14) et le COL4 (Figure 15). La quantité de protéine matricielle (LN5 et COL4) fixe et la quantité de peptide variable sont indiquées sur les graphes (Figures 14 et 15). Les peptides 2 et 3 (décrits dans l'exemple 1) ont été utilisés comme contrôles au cours de cette expérience. Les tests d'adhérence ont été effectués avec des NHK dans les mêmes conditions que dans l'expérience présentée Figure 13 (50 000 cellules par puit). Pour analyser l'effet d'adhérence lié à la présence du peptide, un contrôle sans peptide a été effectué et représente le 100 % d'adhérence (LN5 seule ou COL4 seul). Les résultats ont ensuite été exprimés en pourcentage de ce contrôle.
Comme présenté sur la Figure 14, le peptide TALRIRATYGEY augmente de façon progressive et significative l'adhérence des NHKs à la LN5. L'adhérence à la LN5 est en moyenne doublée pour une quantité de peptide de 0,21 à 0,87 µg/puit. Cet effet est spécifique puisqu'il n'est pas observé pour les peptides contrôles 2 et 3. Les photographies obtenues en microscopie à contraste de phase confirment les résultats quantitatifs et montrent que la LN5 utilisée seule à 0,2 mg induit une adhérence moyenne. L'apport du peptide P1 induit la formation d'un tapis cellulaire confluent alors que les peptides P2 et P3 ne modifient pas l'adhérence obtenue avec la LN5 seule. La même expérience menée avec le COL4 (Figure 15) ne montre pas d'effet significatif du peptide TALRIRATYGEY sur l'adhérence des kératinocytes au COL4.

L'ensemble de ces résultats indiquent que le peptide TALRIRATYGEY potentialise l'adhérence des NHKs à la LN5 lorsque ces 2 protéines sont présentes conjointement.

L'effet du peptide TALRIRATYGEY sur l'adhérence à la LN5 a été vérifié avec des NHKs-âgés (Figure 16). Des NHKs-jeunes (8 ans) ont été testés en parallèle et 30 000 cellules/puit ont été utilisées dans les 2 cas (les NHKs-âgés sont toujours limitant car obtenus en plus faible quantité). D'une manière générale, les résultats obtenus au cours de cette expérience sont moins prononcés que ceux obtenus lors de l'expérience présentée Figure 14 car (1) le nombre de cellules est inférieur (30 000 au lieu de 50 000) et (2) la gamme de peptide testée est également inférieure. Cependant, l'effet de potentialisation du peptide TALRIRATYGEY sur l'adhérence des NHK-âgés à la LN5 est observé. Cet effet, moins prononcé que l'effet obtenu avec les NHKs-jeunes, est vraisemblablement lié à la diminution de l'adhérence des NHKs-âgés au peptide TALRIRATYGEY avec l'âge (Figure 6).

EXEMPLE 5

Détermination de la quantité minimale peptide induisant l'adhérence cellulaire.

**I. Matériel et méthodes**

1°) Production du peptide

Le peptide a été construit, comme décrit dans l'exemple 1. La synthèse peptidique a été effectuée sur un Synthétiseur Milligen 9050 en utilisant une chimie Fmoc-Opfp/Hobt. Le peptide a ensuite été détaché de la résine et déprotégé en utilisant une solution de TFA (acide trifluororacétique) contenant des scavengers (phénol, eau, ethanedithiol et thioanisole). Le peptide a ensuite été analysé et purifié sur une colonne Vydac C18, 5mm de 4.6 ou 10 mm de diamètre et 250 mm de longueur puis caractérisé par spectrométrie de masse électrospray sur un SCIEX API 165.

2°) Détermination de la quantité de peptide immobilisé sur les plaques par la méthode d'analyse des acides aminés.

Le peptide a été dilué dans du PBS stérile. Les échantillons contenant le peptide à doser ont été lyophilisés par évaporation. Ils ont été déposés dans un réacteur et soumis à un vide poussé afin d'éliminer les molécules d'oxygène susceptibles d'oxyder certains acides aminés. L'hydrolyse des liaisons peptidiques a été effectuée à l'aide d'un mélange d'Acide Chlorydrique (HCL 6N, 2/3), Acide Trifluoro-Acétique (TFA, 1/3) dans une enceinte à 150°C pendant 45 minutes. L'hydrolyse en phase gazeuse permet d'éviter les contaminations par des ions présents dans les acides. Les échantillons ont ensuite été séchés et solubilisés dans un tampon approprié à la chromatographie échangeuse d'ions (citrate de sodium). La chromatographie échangeuse d'ions a permis de séparer les acides aminés selon leur force ionique, et à leur sortie de colonne, ils réagissent avec un réactif, la ninhydrine, pour former un complexe coloré avec les amines primaires (violet, lisible à 570 nm). La ninhydrine réagit aussi avec les amines secondaires (proline, hydroxyproline) pour former un composé jaune lisible à 440 nm. Deux chromatogrammes distincts permettent l'analyse des échantillons.

Un analyseur semi-automatique Beckman 6300 optimisé pour cette analyse a été utilisé, associé au logiciel Beckman Gold permettant la quantification des chromatogrammes. Les résultats ont ensuite été transférés sur un tableau MS-Excel sur lequel une macro a permis le calcul des compositions relatives.

3°) Culture des cellules

    a°) Les lignées et kératinocytes humains normaux ( voir Exemple 1)
    b°) Obtention et culture des kératinocytes jeunes et âgés (voir exemple 2)

4') Test d'adhérence cellulaire (voir exemple 2)

**II. Résultats et Discussion**

Détermination de la quantité de peptide immobilisée sur les plaques 96 puits

Les expériences d'adhérence cellulaire effectuées avec le peptide nécessitent une première étape d'immobilisation du peptide sur les plaques de culture 96 puits. Cette étape s'effectue par un contact de la solution contenant le peptide avec la surface de plastique pendant 18 heures à +4°C. Les solutions sont ensuite éliminées par aspirations et le peptide est immobilisé sur la surface, prêt à interagir avec les cellules apportée lors du test d'adhérence cellulaire proprement dit. Selon les propriétés physicochimiques des peptides ou protéines utilisés, un pourcentage plus ou moins important est réellement immobilisé sur le support. Il a donc fallu déterminer avec précision le pourcentage d'immobilisation du peptide TALRIRATYGEY afin de connaître la quantité réelle de peptide inductrice de l'adhérence.

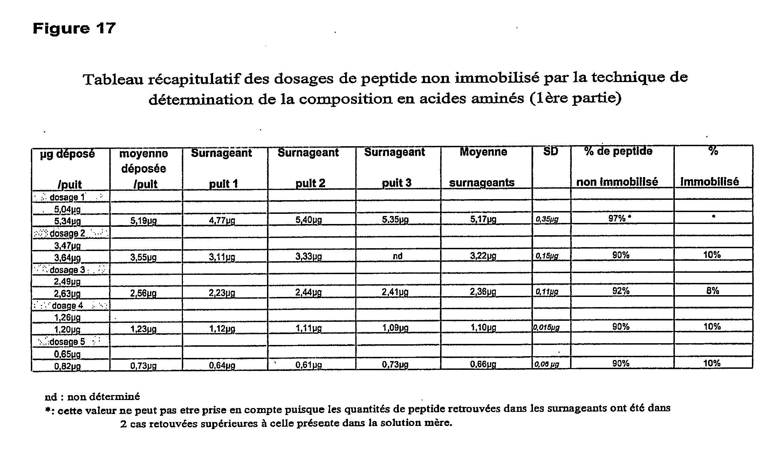

La Figure 17 rassemble les données obtenues lors des expériences de mise au point des conditions de dosage du peptide déposé sur les plaques 96 puits et non immobilisé. Cinq expériences ont été réalisées avec des quantités décroissantes de peptides allant de 5 $\mu$g à 0,7 $\mu$g. Pour chaque condition, la quantité de peptide indiquée a été déposée dans 3 puit différents (100 $\mu$l/puit) d'une plaque 96 puit. Après un contact de 18 h à +4°C, les surnageants ont été recueillis et déposés dans des tubes destinés au dosage par la méthode de détermination de la composition en acide aminés. Deux échantillons identiques de la solution utilisée pour les dépôts ont été re-dosés dans les mêmes conditions. Les premières expériences réalisées avec 5, 19 $\mu$g montrent d'emblée que la quantité de peptide immobilisée est très faible. Les résultats obtenus dans cette condition n'ont pas pu être exploités car les valeurs déterminées dans les surnageants étaient parfois supérieures à celles de la solution de départ. Ceci signifiait d'emblée que la différence entre quantité de départ et surnageant était trop faible pour pouvoir être détectée, et se trouvait certainement comprise dans l'intervalle de l'écart à la moyenne des dosages de la solution de départ. L'expérience a donc été recommencée avec des quantités plus faibles de peptide : 3,55 $\mu$g; 2,56 $\mu$g; 1,23 $\mu$g et 0,73 $\mu$g déposés dans les puits. Les quantités de peptide étaient satisfaisantes pour réaliser l'expérience puisque nous avons obtenu pour ces 4 conditions des résultats équivalents. La quantité de peptide restant dans le surnageant après l'immobilisation est d'approximativement 90 % de la quantité de peptide apporté.

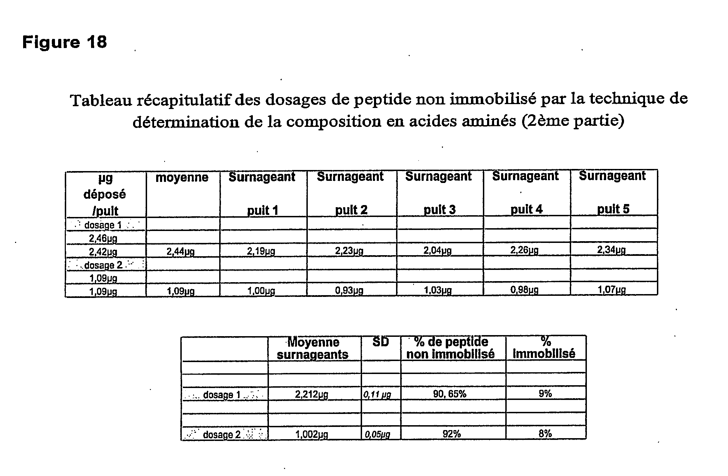

Dans un deuxième temps, 2 quantités de peptides différentes (Q1 et Q2) ont été choisies pour effectuer un dosage sur un plus grand nombre d'échantillons (Figure 18). Deux échantillons de Q1 (2 x 100 $\mu$l) et deux échantillons de Q2 (2 x 100 $\mu$l) ont été directement déposés dans les tubes spéciaux destinés au dosage des deux solutions de départ par la technique de détermination des acides aminés. Cinq puits différents de la plaque 96 puits ont reçu la quantité Q1 (volume 100$\mu$l) et cinq autres puits ont reçu la quantité Q2 (volume 100 $\mu$l). Après un contact de 18 h à +4°C, les 5 surnageants des puits Q1 et les 5 surnageants des puits Q2 ont été recueillis dans des tubes en verre spéciaux pour le dosage des acides aminés. Tous les échantillons, solutions de départ et surnageants ont été dosés en même temps. Le dosage a permis de doser une quantité moyenne de 2,44 $\mu$g dans Q1. Le dosage des 5 surnageants ont permis de déterminer une quantité moyenne de 2,212 $\mu$g étant resté en solution dans les puits, montrant qu'il reste 90, 7 % du peptide dans la solution après l'immobilisation. Ceci signifie que 9,3 % de peptide seulement ont été immobilisés. Un pourcentage équivalent a été retrouvé pour la quantité Q2 dont la quantité moyenne de départ a été de 1,09 $\mu$g. Après l'étape d'immobilisation, le dosage a permis de déterminer la quantité moyenne de 1,002 $\mu$g dans les surnageants ce qui signifie que 92 % de la quantité de peptide n'a pas été immobilisée.

L'ensemble de ces résultats permet de conclure que 8 à 9 % seulement de la quantité de peptide apportée dans le puit au départ est finalement immobilisée.

Cette quantité étant très inférieure à celle apportée dans le puit au départ, ceci permet de déterminer avec précision, la quantité minimale de peptide réellement active.

Pour déterminer la quantité minimale de peptide active, une seconde approche a été utilisée et le nombre de cellules adhérentes a été augmenté dans le but d'obtenir un tapis cellulaire confluent. Les cellules HT1080 ont été choisies pour cette expérience car elles présentent le même profil d'adhérence que les kératinocytes. Les conditions du test d'adhérence sont les mêmes que ce qui a été décrit précédemment. Les courbes d'adhérence au peptide, dans deux conditions d'ensemencement cellulaire, sont présentées par les graphes de la Figure 19. Deux ensemencements cellulaires ont été testés: 80 000 cellules par puit (volume de 100 $\mu$l) et 150 000 cellules par puit (volume 100 $\mu$l). Comme montrées sur la Figure 19, les 2 courbes d'adhérence sont parallèles et atteignent le plateau d'adhérence pour la même quantité de peptide immobilisée. La différence observée entre les deux courbes est liée à la différence d'intensité de l'absorbance qui est supérieure dans le cas où il y a le double de cellules. Des images illustrent sur la droite le tapis cellulaire homogène et confluent obtenu dans le cas où 150 000 cellules ont été ensemencées. Les cellules sont jointives et sont associées les unes aux autres. A noter que le peptide induit l'étalement cellulaire et l'établissement de jonctions cellule-cellule, deux garants d'une bonne communication cellulaire.

A partir de ces conditions, la zone ascendante de la courbe a été analysée pour connaître la quantité minimale de peptide permettant une adhésion significative (Figure 19). L'ensemencement à 150 000 cellules par puit révèle que la quantité minimale de peptide induisant une adhérence significative est située entre 0,008 $\mu$g et 0,015 $\mu$g.

EXEMPLE 6: Exemples de compositions comprenant le peptide TALRIRATYGEY (SEQ ID N°1) :

Composition N°1 : émulsion W/O (eau dans huile)

| Ingrédients | % |
|---|---|
| Aqua(Water) | qs 100 |
| Hexyl Laurate | 37.000 |
| Glycerin | 5.000 |
| Methyl Glucose Isostearate | 3.500 |
| Dimethicone | 3:000 |
| Disteardimonium Hectorite | 3.000 |
| Euphorbia Cerifera ( Candelilla ) Wax | 1.500 |
| PEG-45/Dodecyl Glycol Copolymer | 1.000 |
| Phenoxyethanol, | 0.580 |
| Methylparaben, Ethylparaben, Butylparaben, Propylparaben, Isobutylparaben | 0.420 |
| Chlorphenesin | 0.280 |
| Magnesium Sulfate | 0.100 |
| Disodium EDTA | 0.100 |
| Peptide TARRIRATYGEY (SED ID N°1) | 0.0001 |

Composition N°2 : émulsion O/W (huile dans eau)

| Ingrédients | % |
|---|---|
| Aqua ( Water ) | qs 100 |
| Cyclomethicone | 4.000 |
| Glycerin | 3.000 |
| Hydrogenated Polyisobutene | 3.000 |
| PPG-15 Stearyl Ether | 2.000 |
| Ethylhexyl Palmitate | 2.000 |
| Steareth-2 | 2.000 |
| Steareth-21 | 2.000 |
| Stearic Acid | 1.750 |
| Cetyl Alcohol | 1.500 |
| Phenoxyethanol,Methylparaben, Ethylparaben, Butylparaben, Propylparaben, Isobutylparaben | 0.600 |
| Polyacrylamide, C13-14 Isoparaffin, Laureth-7 | 0.400 |
| Xanthan Gum | 0.300 |
| Chlorphenesin | 0.200 |
| Peptide TALRIRATYGEY (SEQ ID N°1) | 0.001 |

Composition N°3 : lotion

| Ingrédients | % |
|---|---|
| Aqua(Water) | qs 100 |

(suite)

| Ingrédients | % |
|---|---|
| Glycerin | 5.000 |
| Propylene Glycol, Aqua (Water), Benzoic Acid, Phenoxyethanol, Methylparaben, Butylparaben, Ethylparaben, Propylparaben, Isobutylparaben, PEG-40 Hydrogenated Castor Oil | 1.250 |
| Bis-PEG-18 Methyl Ether Dimethyl Silane | 0.500 |
| Tetrasodium EDTA | 0.200 |
| Peptide TALRIRATYGEY (SEQ ID N°1) | 0.0003 |

Composition N°4 : gel

| Ingrédients | % |
|---|---|
| Aqua (Water) | qs 100 |
| Cyclomethicone | 4.000 |
| Glycerin | 3.000 |
| Phenoxyethanol, Methylparaben, Ethylparaben, Butylparaben, Propylparaben, Isobutylparaben | 0.600 |
| Polyacrylamide, C13-14 Isoparaffin, Laureth-7 | 0.500 |
| Carbomer | 0.500 |
| Chlorphenesin | 0.200 |
| Peptide TALRIRATYGEY (SEQ ID N°1) | 0.003 |

**Revendications**

1. Peptide présentant la séquence suivante : TALRIRATYGEY (SEQ ID N°1)

2. Peptide selon la revendication 1 **caractérisé en ce qu'**il est obtenu par synthèse chimique.

3. Composition pharmaceutique **caractérisée en ce qu'** elle comprend au moins un peptide selon l'une quelconque des revendications 1 et 2.

4. Composition pharmaceutique selon la revendication 3, **caractérisée en ce que** la composition contient de 0,00002 % à 5 %, de préférence de 0,00005 % à 0,1 % et plus préférentiellement encore de 0,0001 % à 0,001 % en poids de peptide et au moins un excipient pharmaceutiquement acceptable.

5. Composition pharmaceutique selon la revendication 3 ou 4, **caractérisée en ce qu'**elle comprend en outre au moins un autre principe dermatologiquement actif.

6. Composition pharmaceutique selon l'une quelconque des revendications 3 à 5, **caractérisée en ce qu'**elle se présente sous la forme d'une crème, d'un lait, d'une émulsion huile dans l'eau, d'une émulsion eau dans l'huile, d'une émulsion multiple, d'une solution, d'une suspension, d'un gel aqueux, d'un gel huileux, d'un gel hydroalcoolique, d'une lotion, d'un stick ou d'une poudre.

7. Utilisation du peptide selon l'une quelconque des revendications 1 à 2 pour la préparation d'une composition pharmaceutique destinée à renforcer la jonction dermo-épidermique, l'adhérence cellule-cellule et/ou l'adhérence cellule-matrice au niveau de l'épiderme et à favoriser la réparation de l'épiderme.

8. Utilisation selon la revendication 7, **caractérisée en ce que** ladite composition pharmaceutique est telle que définie dans l'une des revendications 3 à 6.

9. Utilisation selon la revendication 7 ou 8, **caractérisée en ce que** ladite composition est destinée au traitement des altérations de la peau induites par des pathologies dermatologiques.

10. Utilisation selon la revendication 7 ou 8, **caractérisée en ce que** ladite composition est destinée au traitement des altérations de la peau fragilisée par des traitements cosmétiques ou thérapeutiques.

**11.** Utilisation selon la revendication 7 ou 8, **caractérisée en ce que** ladite composition est destinée au traitement curatif ou préventif du vieillissement cutané tel que les rides, le relâchement, la perte d'élasticité, la moins bonne cicatrisation, de la xérose sénile, des modifications du système pigmentaire de la peau, de l'appauvrissement du réseau vasculaire de la peau, et de l'altération des phanères.

**12.** Procédé de traitement cosmétique de la peau **caractérisé en ce qu'**on applique sur la peau une composition cosmétique comprenant au moins un peptide selon l'une quelconque des revendications 1 et 2.

**13.** Procédé de traitement cosmétique de la peau selon la revendication 12 **caractérisé en ce que** ladite composition contient de 0,00002 % à 5 %, de préférence de 0,00005 % à 0,1 % et plus préférentiellement encore de 0,0001 % à 0,001 % en poids de peptide et au moins un excipient cosmétiquement acceptable.

**14.** Procédé de traitement cosmétique de la peau selon l'une quelconque des revendications 12 et 13 **caractérisé en ce que** ladite composition comprend en outre au moins un autre principe cosmétiquement actif.

**15.** Procédé de traitement cosmétique de la peau selon l'une quelconque des revendications 12 à 14 **caractérisé en ce que** ladite composition se présente sous la forme d'une crème, d'un lait, d'une émulsion huile dans l'eau, d'une émulsion eau dans l'huile, d'une émulsion multiple, d'une solution, d'une suspension, d'un gel aqueux, d'un gel huileux, d'un gel hydroalcoolique, d'une lotion, d'un stick ou d'une poudre.

**Claims**

**1.** A peptide with the following sequence: TALRIRATYGEY (SEQ ID NO. 1)

**2.** A peptide according to claim 1 **characterised in that** it is obtained by chemical synthesis.

**3.** A pharmaceutical composition **characterised in that** it comprises at least one peptide according to any of claims 1 and 2.

**4.** A pharmaceutical composition according to claim 3, **characterised in that** the composition contains 0.00002% to 5%, preferably 0.00005% to 0.1 % and most preferably 0.0001 % to 0.001 % by weight of peptide and at least one pharmaceutically acceptable excipient.

**5.** A pharmaceutical composition according to claim 3 or 4, **characterised in that** it further comprises at least one other dermatologically active substance.

**6.** A pharmaceutical composition according to any of claims 3 to 5, **characterised in that** it takes the form of a cream, milk, oil-in-water emulsion, water-in-oil emulsion, multiple emulsion, solution, suspension, water gel, oil gel, water-alcohol gel, lotion, stick or powder.

**7.** Use of the peptide according to any of claims 1 to 2 for the preparation of a pharmaceutical composition designed to reinforce the dermis-epidermis junction, cell-cell adhesion and/or cell-matrix adhesion at the epidermis and favour the repairing of the epidermis.

**8.** Use according to claim 7, **characterised in that** the said pharmaceutical composition is as defined in any of claims 3 to 6.

**9.** Use according to claim 7 or 8, **characterised in that** the said composition is designed for treating changes in the skin induced by dermatological diseases.

**10.** Use according to claim 7 or 8, **characterised in that** the said composition is designed for treating changes in the skin made fragile by cosmetic or therapeutic treatments.

**11.** Use according to claim 7 or 8, **characterised in that** the said composition is designed for the curative or preventive treatment of skin ageing such as wrinkles, slackness, loss of elasticity, diminished healing, senile xerosis, modifications of the skin pigment system, reduction in the density of the skin vascular network and damage to the appendages of the skin.

**12.** A method for the cosmetic treatment of the skin **characterised in that** a cosmetic composition comprising at least one peptide according to any of claims 1 and 2 is applied to the skin.

**13.** A method for the cosmetic treatment of the skin according to claim 12 **characterised in that** the said composition contains 0.00002% to 5%, preferably 0.00005% to 0.1% and most preferably 0.0001% to 0.001% by weight of peptide and at least one cosmetically acceptable excipient.

**14.** A method for the cosmetic treatment of the skin according to any of claims 12 and 13 **characterised in that** the said composition further comprises at least one other cosmetically active substance.

**15.** A method for the cosmetic treatment of the skin according to any of claims 12 to 14 **characterised in that** the said composition takes the form of a cream, milk, oil-in-water emulsion, water-in-oil emulsion, multiple emulsion, solution, suspension, water gel, oil gel, water-alcohol gel, lotion, stick or powder.

**Patentansprüche**

**1.** Peptid mit folgender Sequenz: TALRIRATYGEY (SEQ ID Nr. 1)

**2.** Peptid nach Anspruch 1, **dadurch gekennzeichnet, dass** es durch chemische Synthese erhalten wird.

**3.** Pharmazeutische Zusammensetzung, **dadurch gekennzeichnet, dass** sie mindestens ein Peptid nach einem der Ansprüche 1 und 2 umfasst.

**4.** Pharmazeutische Zusammensetzung nach Anspruch 3, **dadurch gekennzeichnet, dass** die Zusammensetzung zwischen 0,00002 % und 5%, vorzugsweise zwischen 0,00005 % und 0,1 % und am besten zwischen 0,0001 % und 0,001 % an Peptidmasse und mindestens einen pharmazeutisch annehmbaren Trägerstoff umfasst.

**5.** Pharmazeutische Zusammensetzung nach Anspruch 3 oder 4, **dadurch gekennzeichnet, dass** sie zudem mindestens einen anderen dermatologisch aktiven Wirkstoff umfasst.

**6.** Pharmazeutische Zusammensetzung nach einem der Ansprüche 3 bis 5, **dadurch gekennzeichnet, dass** sie in Form einer Creme, einer Milch, einer Öl-/Wasser-Emulsion, einer Wasser-/Öl-Emulsion, einer Mehrfachemulsion, einer Lösung, einer Suspension, eines wässrigen Gels, eines öligen Gels, eines hydroalkoholischen Gels, einer Lotion, eines Sticks oder eines Pulvers vorliegt.

**7.** Anwendung des Peptids nach einem der Ansprüche 1 bis 2 für die Zubereitung einer pharmazeutischen Zusammensetzung zur Stärkung der Verbindung zwischen Leder- und Oberhaut, der Haftung zwischen den Zellen und/oder der Haftung zwischen Zellen und Matrix im Bereich der Oberhaut und zum besseren Regenerieren der Oberhaut.

**8.** Anwendung nach Anspruch 7, **dadurch gekennzeichnet, dass** die pharmazeutische Zusammensetzung der in einem der Ansprüche 3 bis 6 definierten entspricht.

**9.** Anwendung nach Anspruch 7 oder 8, **dadurch gekennzeichnet, dass** diese Zusammensetzung für die Behandlung von Hautveränderungen aufgrund von Hautkrankheiten bestimmt ist.

**10.** Anwendung nach Anspruch 7 oder 8, **dadurch gekennzeichnet, dass** diese Zusammensetzung zur Behandlung von Veränderungen der Haut bestimmt ist, die durch kosmetische oder therapeutische Behandlungen geschwächt ist.

**11.** Anwendung nach Anspruch 7 oder 8, **dadurch gekennzeichnet, dass** diese Zusammensetzung zur heilenden oder vorbeugenden Behandlung von Hautalterungsprozessen wie Falten, nachlassendes straff und elastisch sein, schlechteres Verheilen, senile Xerose, Änderungen des Pigmentsystems der Haut, schlechtere Durchblutung der Haut und Abbau der Hautanhangsorgane bestimmt ist.

**12.** Kosmetisches Behandlungsverfahren der Haut, **dadurch gekennzeichnet, dass** man eine kosmetische Zusammensetzung, die mindestens ein Peptid nach einem der Ansprüche 1 und 2 umfasst, auf die Haut aufträgt.

**13.** Kosmetisches Behandlungsverfahren der Haut nach Anspruch 12, **dadurch gekennzeichnet, dass** diese Zusammensetzung zwischen 0,00002 % und 5 %, vorzugsweise zwischen 0,00005 % und 0,1 % und am besten zwischen 0,0001 % und 0,001 % an Peptidmasse und mindestens einen kosmetisch annehmbaren Trägerstoff enthält.

**14.** Kosmetisches Behandlungsverfahren der Haut nach einem der Ansprüche 12 und 13, **dadurch gekennzeichnet, dass** diese Zusammensetzung zudem mindestens einen anderen kosmetisch aktiven Wirkstoff umfasst.

**15.** Kosmetisches Behandlungsverfahren der Haut nach einem der Ansprüche 12 bis 14, **dadurch gekennzeichnet, dass** diese Zusammensetzung in Form einer Creme, einer Milch, einer Öl-/Wasser-Emulsion, einer Wasser-/Öl-Emulsion, einer Mehrfachemulsion, einer Lösung, einer Suspension, eines wässrigen Gels, eines öligen Gels, eines hydroalkoholischen Gels, einer Lotion, eines Sticks oder eines Pulvers vorliegt.

## FIGURE 1

LE 3    L4    LE 3

⑥⑤④ m 〇 ③②① *N -ter*

GLTKTYTFRLNE
(309-321)

TALRIRATYGEY
(344-355)

I/II

DVKNLENIRDNL
(1169-1180)

*C -ter*

FIGURE 2A

HBL100

peptide 1/lot 1
peptide 1/lot 2
peptide 2
peptide 3

Quantité de peptide / puit (µg)

FIGURE 2B

TALRIRATYGEY (1)

TALRIRATYGEY (2)

DVKNLENIRDNL

GLTKTYTFRLNE

FIGURE 3A.

HT 1080

FIGURE 3B

TALRIRATYGEY (1)    TALRIRATYGEY (2)

DVKNLENIRDNL    GLTKTYTFRLNE

**FIGURE 4A**

**FIGURE 4B**

TALRIRATYGEY (1)       TALRIRATYGEY (2)

DVKNLENIRDNL          GLTKTYTFRLNE

## KERATINOCYTES HUMAINS NORMAUX

FIGURE 5A

Légende:
- peptide 1/lot 1
- peptide 1/lot 2
- peptide 2
- peptide 3

Quantité de peptide / puit (µg)

Adhérence cellulaire (Absorbance à 570 nm)

FIGURE 5B

TALRIRATYGEY (1)     TALRIRATYGEY (2)

DVKNLENIRDNL          GLTKTYTFRLNE

Figure 6

Kératinocytes Humains Normaux

Figure 7

EP 1 784 423 B1

# Figure 8

| | moyenne Abs conc 0 | SD | moyenne Abs conc 0,5% | SD | moyenne Abs conc 1% | SD |
|---|---|---|---|---|---|---|
| 1h | 0,369 | 0,004 | 0,375 | 0,012 | 0,36 | 0,007 |
| 2h | 0,411 | 0,006 | 0,432 | 0,01 | 0,431 | 0,01 |
| 3h | 0,469 | 0,007 | 0,501 | 0,001 | 0,508 | 0,01 |
| 4h | 0,527 | 0,01 | 0,559 | 0,001 | 0,566 | 0,01 |
| 5h | 0,583 | 0,013 | 0,625 | 0,01 | 0,627 | 0,02 |

# Figure 9

Kératinocytes, 10 ans — Absorbance à 490 nm / Temps (heures); courbes 0, 0,05%, 0,1%

Kératinocytes, 71 ans — Absorbance à 490 nm / Temps (heures); courbes 0, 0,05%, 0,1%

| | moyenne Abs conc 0 | SD | moyenne Abs conc 0,05% | SD | moyenne Abs conc 0,1% | SD |
|---|---|---|---|---|---|---|
| 1h | 0,172 | 0,001 | 0,178 | 0,002 | 0,175 | 0,003 |
| 2h | 0,226 | 0 | 0,236 | 0,004 | 0,237 | 0,006 |
| 3h | 0,267 | 0,002 | 0,282 | 0,002 | 0,281 | 0,001 |
| 4h | 0,301 | 0,001 | 0,321 | 0,001 | 0,322 | 0 |
| 5h | 0,345 | 0,005 | 0,365 | 0 | 0,365 | 0,008 |

| | moyenne Abs conc 0 | SD | moyenne Abs conc 0,05% | SD | moyenne Abs conc 0,1% | SD |
|---|---|---|---|---|---|---|
| 1h | 0,15 | 0,002 | 0,158 | 0,002 | 0,159 | 0,003 |
| 2h | 0,205 | 0,002 | 0,216 | 0,001 | 0,217 | 0 |
| 3h | 0,243 | 0,001 | 0,26 | 0,001 | 0,261 | 0,001 |
| 4h | 0,282 | 0,006 | 0,304 | 0,003 | 0,309 | 0,005 |
| 5h | 0,318 | 0,005 | 0,342 | 0,003 | 0,349 | 0,002 |

# Figure 10

## Kératinocytes

| | T | peptide 0.004% | T | peptide 0.013% | T | peptide 0.04% | T | peptide 0.123% | T | peptide 0.37% |
|---|---|---|---|---|---|---|---|---|---|---|
| Abs 1 | 0,648 | 0,741 | 0,925 | 1,263 | 1,077 | 1,3 | 1,008 | 1,361 | 0,915 | 1,273 |
| Abs 2 | 0,706 | 0,688 | 1,124 | 1,128 | 1,39 | 1,365 | 1,235 | 1,237 | | 1,309 |
| Abs 3 | 0,677 | 0,737 | 1,018 | 0,966 | 1,2 | 1,373 | 1,299 | 1,407 | 1,29 | 1,178 |
| Moyenne | 0,677 | 0,722 | 1,02233333 | 1,119 | 1,22233333 | 1,346 | 1,18066667 | 1,335 | 1,1025 | 1,25333333 |
| ecart type | 0,029 | 0,0295127 | 0,09957075 | 0,1487044 | 0,15769063 | 0,040037 | 0,15291937 | 0,087932 | 0,26516504 | 0,06767816 |
| % du temoin | 100% | 106,60% | 100% | 109,70% | 100% | 110% | 100% | 113% | 100% | 113% |

Figure 11

**Figure 12**

Peptide TALRIRATYGEY

0 %    1,25%    0,6%

**Figure 13**

A

Représentation schématique de
la jonction dermo-épidermique

50 000 cellules/puit

C

Laminine 5        Collagène IV

EP 1 784 423 B1

36

# Figure 14

## Kératinocytes Humains Normaux

### 50 000 cellules/puit

Adhérence cellulaire (% du contrôle Laminine 5 seule)

| | |
|---|---|
| ■ | 0 |
| ▨ | 0,21 µg |
| □ | 0,43 µg |
| ▨ | 0,87 µg |
| ▨ | 1,7 µg |

Peptide 1    Peptide 2    Peptide 3

Laminine 5 (0,2 µg) + peptide (quantité en µg)/puit

Laminine 5 (0,2 µg)

Laminine 5 (0,2 µg) + TALRIRATYGEY (µg)

P1 (0,21µg)    P1 (0,43µg)
P1 (0,87µg)    P1 (1,7µg)

Laminine 5 (0,2 µg) + P2 ou P3 (µg)

P1 (0,87µg)    P1 (0,87µg)

Figure 15

Figure 16

**Figure 17**

Tableau récapitulatif des dosages de peptide non immobilisé par la technique de
détermination de la composition en acides aminés (1ère partie)

| µg déposé /puit | moyenne déposée /puit | Surnageant puit 1 | Surnageant puit 2 | Surnageant puit 3 | Moyenne surnageants | SD | % de peptide non immobilisé | % immobilisé |
|---|---|---|---|---|---|---|---|---|
| dosage 1 | | | | | | | | |
| 5,04µg | | | | | | | | |
| 5,34µg | 5,19µg | 4,77µg | 5,40µg | 5,35µg | 5,17µg | 0,35µg | 97% * | * |
| dosage 2 | | | | | | | | |
| 3,47µg | | | | | | | | |
| 3,64µg | 3,55µg | 3,11µg | 3,33µg | nd | 3,22µg | 0,15µg | 90% | 10% |
| dosage 3 | | | | | | | | |
| 2,49µg | | | | | | | | |
| 2,63µg | 2,56µg | 2,23µg | 2,44µg | 2,41µg | 2,36µg | 0,11µg | 92% | 8% |
| doage 4 | | | | | | | | |
| 1,26µg | | | | | | | | |
| 1,20µg | 1,23µg | 1,12µg | 1,11µg | 1,09µg | 1,10µg | 0,015µg | 90% | 10% |
| dosage 5 | | | | | | | | |
| 0,65µg | | | | | | | | |
| 0,82µg | 0,73µg | 0,64µg | 0,61µg | 0,73µg | 0,66µg | 0,06 µg | 90% | 10% |

nd : non déterminé

*: cette valeur ne peut pas etre prise en compte puisque les quantités de peptide retrouvées dans les surnageants ont été dans
2 cas retouvées supérieures à celle présente dans la solution mère.

EP 1 784 423 B1

**Figure 18**

Tableau récapitulatif des dosages de peptide non immobilisé par la technique de détermination de la composition en acides aminés (2ème partie)

| µg déposé /puit | moyenne | Surnageant puit 1 | Surnageant puit 2 | Surnageant puit 3 | Surnageant puit 4 | Surnageant puit 5 |
|---|---|---|---|---|---|---|
| dosage 1 | | | | | | |
| 2,46µg | | | | | | |
| 2,42µg | 2,44µg | 2,19µg | 2,23µg | 2,04µg | 2,26µg | 2,34µg |
| dosage 2 | | | | | | |
| 1,09µg | | | | | | |
| 1,09µg | 1,09µg | 1,00µg | 0,93µg | 1,03µg | 0,98µg | 1,07µg |

| | Moyenne surnageants | SD | % de peptide non immobilisé | % immobilisé |
|---|---|---|---|---|
| | | | | |
| dosage 1 | 2,212µg | 0,11 µg | 90, 65% | 9% |
| | | | | |
| dosage 2 | 1,002µg | 0,05µg | 92% | 8% |

EP 1 784 423 B1

Figure 19

# Figure 20

**HT1080**

| µg | 80 000 cellules Absorbance à 570 nm | d1 | 150 000 cellules Absorbance à 570 nm | d2 |
|---|---|---|---|---|
| 0,0625 | 0,933 | 0,007 | 1,367 | 0,1 |
| 0,0312 | 0,88 | 0,04 | 1,38 | 0,08 |
| 0,0156 | 0,786 | 0,04 | 1,321 | 0,01 |
| 0,008 | | | 0,919 | 0,002 |
| 0,004 | | | 0,194 | 0,06 |
| 0,002 | | | 0,07 | 0,002 |
| 0,0001 | | | 0,031 | 0,004 |
| 0,0005 | | | 0 | 0 |
| 0,00024 | | | 0 | 0 |
| 0,00012 | | | 0 | 0 |
| 0 | 0 | 0 | 0 | 0 |

Adhérence cellulaire (absorbance à 570 nm)

- 150 000 cellules/puit
- 80 000 cellules/puit

µg de peptide immobilisé par puit

EP 1 784 423 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0066731 A, Biostatum
- US 6294356 B1, Jones
- FR 0408383

**Littérature non-brevet citée dans la description**

- **BOYCE ; HAM.** Cultivation, frozen storage, and clonal growth of normal human epidermal keratinocytes in serum free-media. *Tiss Cult. Meth.,* 1985, vol. 9, 83-93